(19) Europäisches Patentamt
European Patent Office
Office européen des brevets

(11) EP 1 567 865 B1

(12) **FASCICULE DE BREVET EUROPEEN**

(45) Date de publication et mention de la délivrance du brevet:
**15.11.2006 Bulletin 2006/46**

(21) Numéro de dépôt: **03789522.4**

(22) Date de dépôt: **28.11.2003**

(51) Int Cl.:
*G01N 33/53* (2006.01) *G01N 33/543* (2006.01)
*G01N 33/58* (2006.01)

(86) Numéro de dépôt international:
**PCT/FR2003/003521**

(87) Numéro de publication internationale:
**WO 2004/051271 (17.06.2004 Gazette 2004/25)**

(54) **PROCEDE DE DETECTION EN CONTINU D'UN ANALYTE, REACTIF TRIFONCTIONNEL DE DETECTION MIS EN OEUVRE ET DISPOSITIF DE DETECTION.**

KONTINUIERLICHE ANALYTEN ÜBERWACHUNG MIT EINER TRIFUNKTIONELLEN REAGENZ

METHOD FOR CONTINUOUS DETECTION OF AN ANALYTE, TRIFUNCTIONAL DETECTING REAGENT USED AND DETECTING DEVICE

(84) Etats contractants désignés:
**AT BE BG CH CY CZ DE DK EE ES FI FR GB GR HU IE IT LI LU MC NL PT RO SE SI SK TR**

(30) Priorité: **28.11.2002 FR 0214959**

(43) Date de publication de la demande:
**31.08.2005 Bulletin 2005/35**

(73) Titulaire: **COMMISSARIAT A L'ENERGIE ATOMIQUE**
**75015 Paris (FR)**

(72) Inventeurs:
- **VOLLAND, Hervé**
  **F-91400 Orsay (FR)**
- **CREMINON, Christophe**
  **F-91400 ORSAY (FR)**
- **NEUBURGER, Laure-Marie**
  **F-92120 Montrouge (FR)**
- **GRASSI, Jacques**
  **F-91440 Bures sur Yvette (FR)**

(74) Mandataire: **Goulard, Sophie et al**
**Cabinet ORES,**
**36,rue de St Pétersbourg**
**75008 Paris Cedex (FR)**

(56) Documents cités:
**EP-A- 0 310 361**
**US-A- 5 183 740**
**US-A- 5 279 943**
**US-A- 5 519 142**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 567 865 B1

Printed by Jouve, 75001 PARIS (FR)

SEQUENCE LISTING

<110> COMMISSARIAT A L'ENERGIE ATOMIQUE

<120> PROCEDE DE DETECTION EN CONTINU D'UN ANALYTE, REACTIF TRIFONCTIONNEL DE DETECTION MIS EN OEUVRE ET DISPOSITIF DE DETECTION

<130> F263PCT86

<150> FR0214959
<151> 2002-11-28

<160> 2

<170> PatentIn version 3.1

<210> 1
<211> 11
<212> PRT
<213> Artificial

<400> 1

```
Arg Pro Lys Pro Gln Gln Phe Phe Gly Leu Met
1               5                   10
```

<210> 2
<211> 15
<212> PRT
<213> Artificial

<220>
<221> MISC_FEATURE
<222> (4)..(4)
<223> lysine biotinylée

<220>
<221> MISC_FEATURE
<222> (4)..(4)
<223> biotinylated lysin

<400> 2

```
Lys Ser Ser Lys Arg Pro Ala Pro Gln Gln Phe Phe Gly Ala Met
1               5                   10                  15
```

## Description

La présente Invention est relative à un procédé de détection en continu en phase hétérogène d'un analyte, au réactif trifonctionnel de détection mis en oeuvre au cours de ce procédé, à son utilisation pour la détection d'un analyte, ainsi qu'au dispositif de détection d'un analyte correspondant.

Il existe à l'heure actuelle de nombreuses techniques permettant la détection d'analytes, et en particulier de molécules dans des conditions de laboratoire: Cependant de plus en plus fréquemment, pour des raisons de santé et de sécurité notamment, il est nécessaire de détecter rapidement la présence de composés qui sont soit des composés d'intérêt (toxines, toxiques chimiques, hormones,...), soit de produits dérivés ou associés avec des molécules d'intérêt, soit de marqueurs d'un événement ou d'une activité particulière (pesticides, métaux lourds, hormones...).

Ces molécules peuvent être présentes dans des milieux très divers tels que l'eau, l'air, la terre, des échantillons biologiques ou bien encore dans des aliments.

Pour une protection et/ou surveillance optimale, la détection de ces molécules doit être effectuée en temps réel et en continu. On entend par dosage en continu un dosage permettant de suivre en permanence la présence ou les modifications de concentration d'une molécule d'intérêt dans un milieu.

Parmi les nombreuses techniques utilisées à ce jour pour détecter ces composés (analytes), un grand nombre utilise la particularité de certaines substances à se lier spécifiquement à ces analytes pour former des complexes. Ces substances que l'on appellera "récepteur", peuvent être de nature très diverse : biologique (anticorps sous forme entière, fragmentaire ou recombinante (Fab', Fab, scFv), récepteurs, acides polynucléiques (ADN ou ARN), acides peptidonucléiques, lectines, protéines transporteuses) ou chimique (chélates, récepteurs synthétiques). Parmi ces substances, l'anticorps présente la plus large utilisation.

De telles méthodes de détection nécessitent un marquage afin de quantifier ou détecter, par un signal, les complexes formés après réaction entre l'analyte à détecter et le récepteur. Ce marquage peut être porté soit par le récepteur soit par une substance (B) correspondant à l'analyte ou à un analogue ou à un fragment de l'analyte ; cette molécule marquée étant souvent appelée traceur.

Suivant que la technique nécessite ou non la séparation des complexes formés des substances libres, on distingue deux grands types de détection dits en phase homogène ou en phase hétérogène.

Les dosages en phase homogène sont réalisés dans un même milieu. Ils sont utilisés lorsque la formation du complexe modifie le signal porté par le récepteur ou la molécule (B) : la formation du complexe peut alors être suivie directement en mesurant le signal. Ces dosages ne nécessitant pas d'étape de séparation permettent une automatisation plus aisée.

Les dosages en phase hétérogène font intervenir, après réaction entre l'analyte et le récepteur, une étape de séparation du traceur non complexé de celui engagé dans les complexes. Cette séparation est souvent réalisée en employant deux milieux ou phases : par exemple une phase solide et une phase liquide.

A partir de ces principes de base, de nombreuses méthodes de dosages ont été décrites, différant notamment selon la nature du marquage utilisé qui peut par exemple être enzymatique, radioactif ou luminescent (Grassi J. *et al,* Handbook of Experimental Pharmacology, Ed. Springer-Verlag, Berlin, 1987, **87,** Chapitre 5; Pelizzola D. *et al,* Q J Nucl. Med., 1995, **39**(4) 251-263).

Parmi ceux-ci, le marquage par un composé luminescent présente l'avantage d'obtenir un signal localisé qui ne nécessite pas la présence d'autres réactifs comme c'est le cas pour les marquages enzymatiques. Ce type de marquage permet également l'utilisation de phénomène tel que le transfert d'énergie qui peut s'effectuer selon différents mécanismes : transfert d'énergie par résonance, transfert d'énergie radiatif (l'accepteur absorbe la lumière émise par le donneur), transfert d'électron (Matko J. *et al,* Biochemistry, 1992, **31,** 703-711 ; Nikoobakht B. *et al,* Photochemistry and Photobiology, 2002, 75, 591-597).

Ce transfert d'énergie, entre un composé "donneur" (D) luminescent et un composé "accepteur" (A) luminescent ou non, et qui est dépendant de la distance entre A et D, a été utilisé pour la réalisation de nombreux dosages. On choisit D et A, qui sont couplés à l'accepteur ou l'analyte, afin que le transfert d'énergie n'ait lieu que lorsque le complexe récepteur/analyte est formé. Ce phénomène se traduit par une diminution ou extinction de la luminescence de D et une émission de luminescence de A si celui-ci est luminescent, lorsque D est excité. Lors de ces dosages on mesure soit la variation de la luminescence de A, soit la variation de la luminescence de D ; la nature de A et de D étant variable.

C'est ainsi que différents Auteurs ont déjà proposé des méthodes de détection d'un analyte par mesure de la luminescence de A :

- la méthode décrite par exemple par Arai R. *et al.* (Protein Engineering, 2000, **13**(5), 369-396) utilise comme donneur et accepteur deux protéines fluorescentes. Ces deux protéines sont produites par biologie moléculaire, chacune formant une protéine chimère avec soit la région variable de la chaîne lourde soit la région variable de la chaîne légère d'un même anticorps. La présence de la molécule contre laquelle est dirigé l'anticorps induit la formation d'un complexe avec les deux protéines chimériques. La distance entre les deux protéines fluorescentes est alors

compatible avec un transfert d'énergie, l'excitation de D va donc induire une fluorescence de A qui sera mesurée ;

- selon la demande internationale WO 96/42016, il est possible d'utiliser comme donneur un complexe de terres rares (europium, terbium) avec un chélate, un cryptate ou un macrocycle et comme accepteur une protéine fluorescente. A et D peuvent être, soit couplés à deux récepteurs pouvant se complexer simultanément sur l'analyte et la présence de ce dernier se traduit alors par une émission de luminescence de A lorsque D est excité, soit A est couplé à un récepteur et D à l'analyte et la présence de l'analyte va induire une compétition avec l'analyte couplé à D pour la complexation avec le récepteur (dosage compétitif), la luminescence de A devenant alors inversement proportionnelle à la quantité de l'analyte ajouté. Ce type de dosage est appelé dosage par luminescence résolue dans le temps.

D'autres Auteurs ont également proposé des méthodes de détection d'un analyte par mesure de la luminescence de D. Ces dosages utilisent la capacité d'un composé (A) à diminuer ou supprimer la luminescence d'un autre composé (D) lorsque ceux-ci sont suffisamment proches ("Quench"). La gamme de molécules A pouvant être utilisées est donc plus étendue et inclut ainsi des composés non luminescents tels que des métaux lourds, des atomes lourds, des molécules chimiques comme par exemple le rouge de méthyle, des nanoparticules telles que celles vendues sous la dénomination Nanogold® par la société Nanoprobes (USA), ou bien encore les molécules vendues sous les dénominations DABCYL® (Eurogentec, Belgique), QSY Dyes (Molecular Probes Inc., USA), ElleQuencher® (Oswell/Eurogentec) ou Black Hole Quenchers® (Biosearch Technologies Inc., USA).

A titre d'exemples :

- le brevet américain n° 3,996,345 décrit une méthode mettant en oeuvre des paires de molécules fluorescente/"Quencher" dans des dosages utilisant des anticorps comme récepteurs ;
- la méthode proposée par Adamczyk M *et al.* (Organic Letters, 2001, 3, 1797-1800) utilise une protéine bioluminescente (Aequorine®) comme donneur et du QSY-7 ou du DABCYL® comme accepteur ou "Quencher". D est couplée à une molécule de biotine et A à une molécule d'avidine. La formation du complexe biotine/avidine provoque une diminution de la bioluminescence de D, la présence de biotine libre ou couplée à une protéine va induire une compétition avec la biotine couplée à D pour la complexation avec l'avidine, la bioluminescence de D sera alors proportionnelle à la quantité de biotine ajoutée ;
- Lee M. *et al.* (J. Agr. Food Chem., 1999, **47**, 2766-2770) et Schobel *et al.* (Bioconjugate Chem., 1999, **10**, 1107-1114) décrivent l'utilisation de deux molécules fluorescentes comme donneur et "Quencheur". Le donneur est couplé à un anticorps et l'accepteur est couplé à l'analyte. Comme précédemment la formation du complexe anticorps-D/analyte-A provoque une diminution de la fluorescence de D, qui est restaurée par la présence de l'analyte dans le milieu ;
- Dubertret B. *et al.* (Nature Biotechnology, 2001, **19**, 365-370) et Bonnet *et al.* (Proc. Natl. Acad. Sci., 1999, **96**, 6171-6176) décrivent l'utilisation d'un acide polynucléique ("Beacon") qui à la particularité de s'hybrider avec lui-même en se repliant. Selon la méthode décrite dans cet article un composé fluorophore (D) est fixé à l'extrémité 3' de l'acide polynucléique et un accepteur (A : nanoparticule ou DABCYL®) est fixé à l'extrémité 5'. Les deux extrémités du "Beacon" étant proches, la fluorescence de D est diminuée par A. La présence d'un acide polynucléique (analyte) pouvant s'hybrider avec une région du "Beacon" va induire une linéarisation de ce dernier et donc une augmentation de la distance entre D et A, et ainsi diminuer l'atténuation de la fluorescence de D par A ;
- le brevet américain n° 5,279,943 propose une méthode de détection d'un analyte dans laquelle des atomes lourds sont utilisés comme "quenchers" de la luminescence ;
- le brevet américain n° 5,229,302 décrit un procédé de détection d'un analyte dans un milieu dans lequel un composé chimique est couplé à un ligand ou à un analogue de celui-ci qui, lorsque le complexe anticorps/ligand est formé, atténue la fluorescence émise par l'anticorps lors d'une excitation à 280 nm. La présence de l'analyte dans le milieu va induire une compétition pour la complexation avec l'anticorps et ainsi restaurer la fluorescence de l'anticorps ;
- Chen C.T. *et al.* (Science, 1998, **279**, 851-852) ont décrit une méthode de détection d'un analyte dans un milieu au moyen d'un récepteur synthétique sur lequel est fixé un fluorophore et un quencher. En absence d'analyte le quencher est suffisamment proche du fluorophore pour diminuer sa fluorescence. La complexation de l'analyte sur le récepteur va augmenter la distance entre le quencher et le fluorophore et ainsi augmenter la fluorescence de ce dernier.

Il est à noter que l'ensemble des dosages utilisant le transfert d'énergie décrits précédemment sont des dosages en phase homogène.

Or, dans le cadre d'une détection en continue d'un analyte, les dosages en flux semblent particulièrement adaptés car ils permettent un apport permanent en échantillon.

C'est ainsi que plusieurs types de dosage en flux ont déjà été décrits :

- plusieurs Auteurs ont notamment proposé des procédés de dosage d'analytes mettant en oeuvre un anticorps

immobilisé sur les parois d'un capillaire, cet anticorps étant saturé par un analyte marqué ou par un analogue marqué de celui-ci. Le passage de l'analyte dans le capillaire induit une compétition avec J'analyte marqué pour la liaison avec l'anticorps. Ainsi, une certaine quantité de l'analyte marqué va être libérée et détectée en sortie du capillaire (voir notamment les brevets américains n° 5,183,740 et 6,323,041 et Sheikh *et al.* Biosensor & Bioelectronics, 2001, **16**, 647-652) ;

- à l'inverse, Barzen C. *et al* (Biosensor & Bioelectronics, 2002, **17**, 289-295) ont décrit un procédé de détection d'un analyte dans lequel ils utilisent une phase solide sur laquelle est immobilisé l'analyte ou un analogue de l'analyte à détecter. L'échantillon contenant ou non l'analyte à détecter est préincubé avec un anticorps anti-analyte marqué. Lors du passage de cette solution sur la phase solide, les anticorps non-complexés vont se fixer sur la phase solide *via* l'analyte immobilisé. Ainsi le signal fluorescent mesuré au niveau de la phase solide est inversement proportionnel à la quantité d'analyte présent dans l'échantillon ;
- Plowman T.E., *et al.* (Anal. Chem., 1999, **71**, 4344-4352) ont développé une méthode de dosage permettant de doser simultanément, sur une même phase solide, différents analytes. Selon cette méthode, des anticorps reconnaissants différents analytes sont immobilisés sur une phase solide à des endroits distincts. Une solution, contenant l'échantillon (avec les différents analytes) et différents anticorps anti-analytes marqués à l'aide d'un fluorophore, est déposée sur la phase solide. Enfin, on mesure la fluorescence qui est issue de la complexation des anticorps de capture, de l'analyte et des anticorps traceurs correspondant aux différents sites d'immobilisation des anticorps. Comme pour le dosage précédent la nécessité de mélanger l'analyte avec un anticorps marqué rend ce procédé difficilement applicable à un dosage en continu car cela impliquerait un apport permanent en réactif ;
- Ligler F.S. *et al.* (Environnemental Science & Technology, 1998, **32**, 2461-2466) ont développé un biocapteur permettant de détecter des molécules présentes dans l'air. Ce biocapteur est constitué d'une fibre sur laquelle sont couplées des molécules d'avidine. Des anticorps anti-analytes biotinylés sont immobilisés sur cette fibre *via* le complexe avidine/biotine. La détection s'effectue en passant l'échantillon (molécules présentent dans l'air, solubilisées dans un tampon) sur cette fibre puis des anticorps anti-analytes marqués avec un fluorophore. Enfin la fluorescence, issue de la complexation des anticorps de capture, de l'analyte et des anticorps traceurs, est mesurée. Cependant, les différentes étapes nécessaires à la réalisation de ce procédé et l'apport en anticorps marqués qui devrait être permanent ne permettent pas une réelle détection en continue.

Il ressort donc de la description des différents procédés de détection connus de l'art antérieur, que tous les dosages en flux disponibles à ce jour sont des dosages en phase hétérogène (phase solide/phase liquide). En effet des dosages en phase homogène en flux nécessiteraient l'apport en continu de récepteurs et/ou d'analytes marqués, ce qui serait coûteux et plus difficile à mettre en oeuvre.

Enfin, il a également déjà été proposé, notamment dans le brevet américain n° 5,976,896 l'utilisation de capillaires pour effectuer des dosages d'analytes en phase hétérogène selon un procédé pouvant être réalisé conformément à plusieurs variantes :

- selon une première variante de ce procédé, un premier récepteur de l'analyte à détecter est immobilisé à la surface intérieure du capillaire. L'échantillon contenant l'analyte est préincubé avec un second récepteur (ne possédant pas le même site de fixation sur l'analyte que le premier récepteur fixé sur le capillaire) marqué avec un composé fluorescent. Cette solution, après réaction, est introduite dans le capillaire, le complexe analyte/récepteur marqué va alors se fixer sur la surface du capillaire en se liant au premier récepteur. Après lavage, le signal fluorescent, qui est proportionnel à la quantité d'analyte dans l'échantillon, est mesuré ;
- selon une deuxième variante de ce procédé, un analogue de l'analyte est immobilisé à la surface intérieure du capillaire. L'échantillon contenant l'analyte à détecter, préalablement préincubé avec un récepteur de l'analyte marqué avec un composé fluorescent, est introduit dans le capillaire. Il va se produire une compétition entre l'analyte fixé sur la surface du capillaire et l'analyte éventuellement présent dans l'échantillon pour la fixation sur le récepteur marqué. Après lavage, le signal fluorescent, qui est inversement proportionnel à la quantité d'analyte présent dans l'échantillon, est mesuré ;
- selon une troisième variante de ce procédé, un récepteur de l'analyte à détecter est immobilisé à la surface intérieure du capillaire. L'échantillon contenant l'analyte préalablement mélangé avec un analyte marqué ou un analogue de cet analyte marqué avec un composé fluorescent est introduit dans le capillaire. Il va alors se produire une compétition entre le composé marqué et J'analyte de l'échantillon pour la fixation sur le récepteur fixé à la surface du capillaire. Après lavage, le signal fluorescent, qui est inversement proportionnel à la quantité d'analyte dans l'échantillon, est mesuré.

Les différentes étapes de ces dosages (incubations, lavages) et la nécessité de mélanger l'analyte avec un composé marqué rend difficilement applicable ce dosage à un dosage en continu car cela impliquerait un apport permanent en réactif.

Par ailleurs, l'ensemble des procédés de détection d'analytes en phases hétérogènes précédemment décrits nécessite le plus souvent la réalisation d'une étape distincte de régénération de la phase solide avant de pouvoir effectuer une nouvelle détection de l'analyte au sein d'un nouvel échantillon. Cette étape limitante de régénération de la phase solide interdit par conséquent l'application de ces différents procédés à la détection en continu d'un analyte dans un milieu.

C'est donc afin de remédier à l'ensemble de ces inconvénients et de pourvoir en particulier à un procédé de détection en phase hétérogène d'un analyte, qui soit simple à mettre en oeuvre, automatisable et au cours duquel l'étape de régénération de la phase solide puisse être réalisée simultanément à l'étape de détection de l'analyte proprement dite sans interrompre cette dernière, que les Inventeurs ont mis au point ce qui fait l'objet de la présente Invention.

Les Inventeurs se sont également donnés pour but de pourvoir à un nouveau réactif utilisable dans des procédés de détection d'analytes en phase hétérogène permettant d'éviter toute incubation, préalablement à l'étape de détection proprement dite, de l'analyte avec un anticorps marqué ou un analyte marqué.

La présente Invention a donc pour premier objet un procédé de détection d'un analyte a dans un échantillon fluide, caractérisé par le fait qu'il comprend les étapes suivantes :

1) la saturation d'un support solide comportant, sur au moins une partie de sa surface, au moins un réactif trifonctionnel (tripode Y) comportant les trois pôles fonctionnels suivant :

   i) un groupement luminescent (L),
   ii) une molécule (B) choisie parmi l'analyte a, un analogue de l'analyte a ou un fragment de l'analyte a; et
   iii) une fonction assurant la fixation dudit réactif trifonctionnel sur la surface dudit support solide ;

par un récepteur de l'analyte a, ledit récepteur étant marqué par un composé accepteur (Q) (récepteur-Q) de la luminescence du groupement L, pour former un complexe C entre ladite molécule (B) et ledit récepteur-Q ;
2) la mise en contact du support solide obtenu à l'étape 1) avec un échantillon fluide susceptible de renfermer l'analyte a à détecter ;
3) la mesure de l'intensité du signal émis par le groupement L qui est proportionnelle à la quantité d'analyte a présent au sein de l'échantillon fluide ; et
4) la régénération du support solide par mise en contact dudit support solide avec du récepteur-Q.

Selon ce procédé, l'étape 1) permet la complexation de la molécule (B) avec le récepteur-Q. A l'issue de cette première étape la luminescence de L est diminuée ou supprimée. Lors de l'étape 2), la mise en présence de l'échantillon avec la surface du support solide va induire, lorsque ledit échantillon renferme l'analyte a, une compétition entre l'analyte a et la molécule (B) pour la formation du complexe avec le récepteur-Q. La liaison de l'analyte a sur le récepteur-Q va provoquer l'élimination du récepteur-Q de la surface du support solide et restaurer la luminescence émise par le composé (L) présent sur le tripode Y. L'intensité du signal mesuré lors de l'étape 3) est alors proportionnelle à la quantité d'analyte a présente dans l'échantillon à analyser. L'étape 4) de régénération va entraîner à nouveau la complexation du récepteur-Q sur le tripode Y et ainsi la suppression de la luminescence de L pour permettre une nouvelle détection de l'analyte a au sein d'un nouvel échantillon.

Le principe du procédé de détection d'un analyte a conforme à l'Invention est schématisé sur le Schéma A ci-après :

## SCHÉMA A

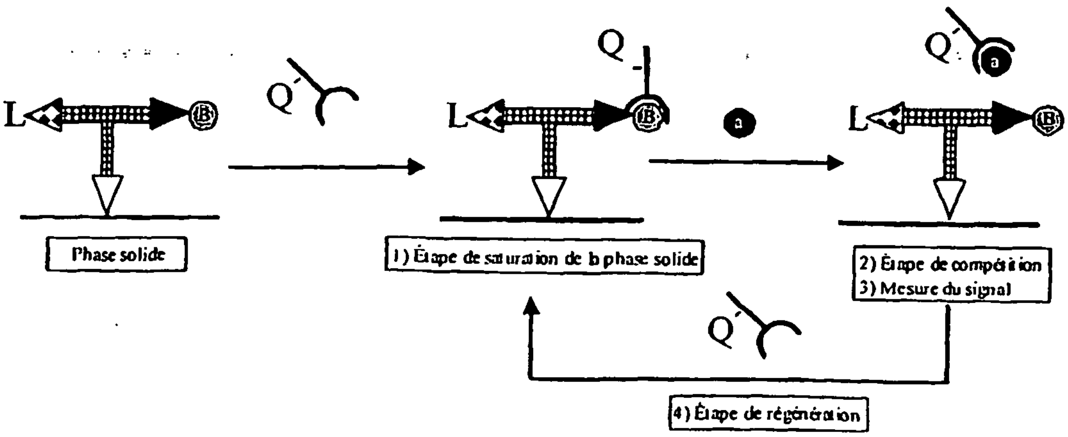


Le procédé de détection conforme à l'Invention présente un grand nombre d'avantages:

1) grâce en particulier à la conformation structurelle particulière du tripode Y mis en oeuvre au cours du procédé, l'étape de régénération de la phase solide peut être réalisée très facilement, et ce sans altération de ses propriétés. En effet, dans tous les dosages en flux précédemment décrits, la mesure du signal s'effectue après formation d'un complexe lié à la phase solide. Par conséquent, et même si certains des procédés de dosage décrits par l'état antérieur de la technique permettent plusieurs dosages successifs (Sheikh *et al.* et brevets américains n° 5,183,740 et 6,323,041 ; pré-cités), ceux-ci sont néanmoins en nombre limité, et nécessitent une étape de régénération du support solide souvent longue et contraignante, qui se traduit par la dissociation du complexe formé. De plus, les conditions drastiques de réalisation de ces étapes de régénération (passage de solutions acides ou basiques) imposent l'arrêt du procédé de détection interdisant ainsi toute application de ces procédés à des dosages en continu d'un analyte donné.
Par contre, selon le procédé de détection conforme à l'Invention, la présence de l'analyte a dans l'échantillon entraîne la dissociation entre le récepteur-Q et la molécule (B), l'étape de régénération consiste donc tout simplement à reformer ce complexe en ajoutant du récepteur-Q. Cette étape de régénération n'implique donc pas l'utilisation de solutions acides ou basiques pouvant altérer les propriétés des molécules de la phase solide ou une réaction d'échange entre deux molécules au niveau du site de liaison du récepteur dont la cinétique est plus longue que la réaction de formation d'un complexe.
2) le signal mesuré au niveau de la région sur laquelle a été immobilisé le tripode Y ce qui permet d'obtenir un signal localisé, contrairement aux dosages développés selon l'art antérieur, notamment par Sheikh *et al.,* ainsi que dans les brevets américains n° 5,183,740 et 6,323,041 pré-cités, dans lesquels le signal, lié à des molécules en solution, est mesuré à la sortie d'un capillaire.
3) le signal étant localisé, plusieurs molécules pourront être détectées simultanément sur le même support solide en fixant sur des zones distinctes et connues de celui-ci plusieurs types de tripodes Y différant les uns des autres par la nature de la molécule (B) qu'ils comprennent.
4) le signal mesuré correspond à la totalité des molécules d'analyte a ayant été en contact avec le support solide entre deux régénérations. Cette particularité du procédé conforme à l'Invention autorise un suivi permanent tout en effectuant des mesures espacées dans le temps.
5) ce format de dosage est applicable à l'ensemble des molécules car il ne requiert pas la liaison simultanée de deux récepteurs sur l'analyte comme cela est quelque fois nécessaire selon les procédés de dosage antérieurement connus comme par exemple dans le procédé décrit par Plowman T.E. *et al.,* pré-cité) et qui nécessitent une taille suffisante de l'analyte.
6) la présence de l'analyte a dans l'échantillon se traduit par une apparition de signal contrairement à la plupart des dosages par compétition connus de l'art antérieur, l'apparition d'un signal autorisant une détection plus aisée.
7) enfin, le système de détection utilisant le phénomène de transfert d'énergie, il permet également de détecter et de quantifier la présence d'un analyte a par la variation de la luminescence du composé Q s'il est fluorescent ou la variation du temps apparent de décroissance de la luminescence du composé (L).

Selon une forme de réalisation particulièrement préférée du procédé conforme à l'Invention, les étapes 3) de

mesure de l'intensité du signal émis et 4) de régénération de la surface du support solide sont réalisées en continu.

Selon l'Invention, le support solide est de préférence choisi parmi les matériaux comportant à leur surface, de façon naturelle ou après modification, des fonctions aptes à former une liaison de nature covalente avec une fonction complémentaire du tripode Y. Ces fonctions peuvent notamment être des fonctions hydroxyle, amine, sulfhydryle ou carboxyle.

Parmi de tels matériaux, on peut en particulier citer les verres, les plastiques (par exemple le polystyrène), les céramiques (de préférence de type oxyde), les métaux (par exemple l'aluminium ou l'or) et les métalloïdes (tel que le silicium oxydé).

De tels supports peuvent notamment se présenter sous la forme de tubes, de capillaires, de plaques telles que des microplaques, de billes ou sous toute autre forme appropriée à la réalisation du procédé conforme à l'Invention.

L'échantillon fluide contenant ou non l'analyte a à détecter peut être de nature ou d'origine diverses telles que par exemple de l'eau, un milieu biologique liquide,ou bien encore un liquide contenant des molécules gazeuses dissoutes ou provenant d'échantillons solides.

La mesure de l'intensité du signal émis lors de l'étape 3) peut être effectuée par un détecteur de luminescence tel que par exemple un fluorimètre.

Selon le procédé conforme à l'Invention, le complexe C formé à l'issue de l'étape 1) de saturation est de préférence choisi parmi les complexes de formule (I) suivante :

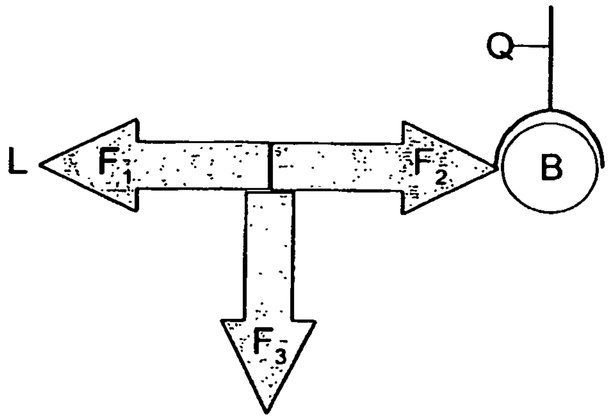

(I)

dans laquelle :

- les flèches représentent la structure du squelette du tripode Y qui est un bras de liaison constitué par une chaîne peptidique, nucléotidique, glucosidique

ou par une chaîne hydrocarbonée linéaire ou ramifiée, saturée ou insaturée ; lesdites chaînes étant éventuellement substituées, interrompues et/ou terminées par un ou plusieurs hétéroatomes tels que N, O ou S et/ou par un ou plusieurs acides aminés, et comportant trois fonctions chimiques réactives $F_1$, $F_2$ et $F_3$ ;

- L représente un groupement luminescent lié de façon covalente au tripode Y par l'intermédiaire de la fonction chimique réactive $F_1$;
- B représente un analyte a, un analogue structurel d'un analyte a ou un fragment d'un analyte a sur lequel est fixé, de façon non covalente et réversible, un récepteur spécifique de l'analyte a, ledit récepteur étant marqué par un composé Q; la molécule (B) étant liée de façon covalente au tripode Y par l'intermédiaire de la fonction chimique réactive $F_2$ ;
- Q représente un composé accepteur de la luminescence du groupement L ;
- $F_3$ représente une fonction chimique réactive apte à permettre la fixation du tripode Y sur la surface du support solide.

Selon l'Invention et indépendamment l'une de l'autre, les fonctions $F_1$, $F_2$, $F_3$ assurent :

i) soit une liaison directe *via* une fonction chimique correspondante présente sur le composé luminescent, la molécule (B) ou la phase solide ;

ii) soit une liaison indirecte. Dans ce deuxième cas, la liaison peut être réalisée en couplant à l'une au moins des fonctions $F_1$, $F_2$ et/ou $F_3$, une molécule $M_1$ apte à former un complexe avec une molécule $M_2$ préalablement fixée sur au moins une partie de la surface de la phase solide, sur la molécule (B) et/ou sur le groupement luminescent.

A titre d'exemple, on peut notamment coupler de la biotine ou de la streptavidine (ou neutravidine ou avidine) sur l'une des fonctions $F_1$, $F_2$ et $F_3$ et respectivement de la streptavidine (ou neutravidine ou avidine) ou de la biotine sur la phase solide, sur la molécule (B) et/ou sur le composé luminescent. Selon une autre variante du procédé conforme à l'Invention, cette liaison indirecte peut également s'effectuer *via* une molécule $M_3$, préalablement couplée sur la phase solide avec un ou plusieurs composés luminescents ou molécules (B) et qui sera couplée à la fonction $F_1$, $F_2$ ou $F_3$. A titre d'exemple de molécules $M_3$, on peut notamment utiliser des protéines telles que l'albumine et la polylysine, des nucléotides, des sucres ou encore d'autres dérivés synthétiques.

De préférence, $F_1$, $F_2$ et $F_3$, identiques ou différentes, sont choisies parmi les fonctions thiols ; amines ; alcools ; acides tels que les fonctions acide carboxylique; esters tels que les esters activés comme par exemple les esters succinimydiliques et les anhydrides ; isothiocyanates ; isocyanates ; acylazides ; chlorures de sulfonyle ; aldéhydes ; glyoxals ; époxydes ; oxiranes ; carbonates ; imidoesters ; carbodiimides ; maléimides ; nitriles, aziridines ; acryloyl ; les dérivés halogénés ; les groupements disulfides ; phosphorées tels que par exemple les phosphates, phosphonates, phosphines et phosphites ; diazo ; carbonyldiimidazole ; hydrazides ; arylazides ; hydrazines ; diazirines ; magnésiens ; lithiens ; cuprates ; zinciques et les systèmes insaturés.
Parmi ces différentes fonctions et groupements, on peut en particulier citer les fonctions amines telles que celles de formules $R-NH_2$, R-NH-, $(R)_3-N$, R-NH-OR et $NH_2-OR$ ; les fonctions alcool R-OH ; et les groupements halogénés de formule R-X avec X représentant un atome d'halogène tel que le chlore, l'iode, le brome ou le fluor ; étant entendu que dans lesdites formules R représente un radical, alkyle, de préférence en $C_1-C_{15}$, aryle, vinyle, ou allyle.
Selon l'Invention, on entend par groupement aryle, tout groupement aromatique possédant un ou plusieurs noyaux benzéniques, naphtalénique ou anthracéniques, lesdits noyaux contenant éventuellement un ou plusieurs hétéroatomes tels que O, N ou S, et étant éventuellement substitués par un ou plusieurs groupes choisis parmi les atomes d'halogène, les radicaux alkyles en $C_1-C_4$, amino, aminoalkyle en $C_1-C_4$, alkyl($C_1-C_4$)aminoalkyle en $C_1-C_4$, dialkyle ($C_1-C_4$)aminoalkyle en $C_1-C_4$, nitro, alkylèneamino en $C_1-C_4$ ou alkénylèneamino en $C_2-C_4$.
Parmi de tels groupements aryle, on peut en particulier citer les groupements benzyle, phényle, crésyle, toluyle, pyridine, pyrimidine et pyrazine.
Selon l'Invention, on entend par groupement luminescent toute substance qui, quand elle est excitée à une longueur d'onde donnée ou par un composé chimique donné, est capable d'émettre un photon, par exemple fluorophore ou terre rare.
Parmi de tels groupements luminescents, on peut notamment citer la fluorescéine (fluorescéinate de sodium) et ses dérivés tels que l'isothiocyanate de fluorescéine (FITC) ; la rhodamine et ses dérivés tels que la tetraméthyl rhodamine isothiocyanate (TRITC) ; le diaminidophényl indo (DAPI) ; l'acridine ; les colorants fluorescents à amines réactives tels que l'ester succinimidylique de l'acide 6-((7-amino-4-méthylcoumarin-3-acétyl)amino) hexanoïque (AMCA) ; les colorants fluorescents vendus sous les dénominations commerciales BODIPY® tels que BODIPY® FR-$Br_2$, BODIPY® R6G, BODIPY® TMR, BODIPY® TR et les BODIPY® 530/550 (longueur d'onde d'excitation/longueur d'onde d'émission, en nm), 558/56/, 564/570, 576/589, 581/591, 630/650 et 650/665 vendus par la société Bio-Rad Inc. (USA), les colorants Cascade Blue (Trilink BioTechnologies (USA), Cy2, Cy3, Cy3.5, Cy5, Cy5.5, et Cy7 (Bio-Rad Inc., USA), DABCYL® et EDANS® (Eurogentec, BE) ; l'Eosine ; l'Erythrosine ; le 6-Fam et le Texas Red.
Selon l'Invenlion, on entend par "récepteur" toute substance apte à former une liaison non covalente et réversible (complexe) avec l'analyte, un analogue de l'analyte ou un fragment de ce dernier. Ce récepteur est bien évidemment choisi en fonction de la nature de la molécule (B) du tripode Y.
Ces récepteurs peuvent notamment être choisis parmi les composés de nature biologique (anticorps sous forme entière, fragmentaire ou recombinante (Fab', Fab, scFv), récepteurs, acides polynucléiques (ADN ou ARN), acides peptidonucléiques, lectines ou bien encore protéines transporteuses) et les composés de nature chimique tels que par exemple les chélates et les récepteurs synthétiques spécifiques.
Parmi de tels récepteurs, on peut par exemple citer les anticorps monoclonaux anti-Substance P anti-protéine prion, anti-angiotensine 11, la poly-Histidine, le système nitrilo-acide triacétique-Nickel (NTA-Nickel), les sondes nucléotidiques complémentaires.
Selon une variante préférée du procédé de détection conforme à l'Invention, ledit récepteur présente une plus grande affinité pour l'analyte a que pour la molécule (B).
Selon l'Invention, on entend par "composé accepteur" (Q), toute molécule permettant la diminution ou la disparition de la luminescence du composé luminescent (L) lorsque le récepteur est complexé à la molécule (B). Ce composé, de natures diverses, peut notamment être un composé chimique (luminescent ou non), un atome lourd ou une nanoparticule.
Parmi de tels composés (Q), on peut en particulier citer les composés fluorescents tels que ceux cités ci-dessus pour les groupements L, la rhodamine et ses dérivés tels que la tetraméthyl rhodamine (TMR), des molécules non-fluorescentes telles que les composés vendus sous les dénominations commerciales Black Hole Quencher® 1, 2 et 3 (Biosearch Technologies), Nanogold Particules® (Nanoprobes), Eclipse Dark Quencher® (Epoch Bioscience), Elle

Quencher® (Oswell), le vert de malachite, et les colorants QSY®7, QSY®9 et QSY®21 (Molecular Probes).

Lorsque la molécule (B) est un peptide ou un oligonucléotide, alors le tripode Y utilisé selon le procédé conforme à l'Invention peut être préparé en effectuant une synthèse peptidique ou oligonucléotidique au cours de laquelle on ajoute à ladite molécule (B) au moins un acide aminé (ou nucléotide modifié) comportant une fonction ($F_1$) et un autre acide aminé (ou nucléotide modifié) comportant une fonction ($F_3$), $F_1$ et $F_3$ ayant les mêmes significations que celles indiquées ci-dessus. Dans ce cas, $F_1$ et $F_3$ assurent également la liaison respectivement avec le composé luminescent (L) et la surface de la phase solide. Tout comme précédemment, les acides aminés (ou nucléotides) comportant les fonctions chimiques $F_1$ et $F_3$ peuvent également être substitués par un acide aminé (ou nucléotide) couplé à une biotine (par exemple le produit 9-fluorénylméthoxycarbonyl (Fmoc)-Lysine(Biotine)-OH, vendu par la société Calbiochem-Novabiochem AG). Dans ce cas, cet acide aminé (ou nucléotide) assurera la liaison avec la surface de la phase solide ou avec le composé luminescent (L) sur lesquels on aura préalablement couplé de la streptavidine (ou neutravidine ou avidine) pour former une liaison indirecte.

Parmi les complexes C de formule (I) conformes à l'Invention, on peut notamment citer les composés dans lesquels :

i) (B) est choisi parmi les peptides, les protéines, les oligonucléotides, les sucres et les acides peptidonucléiques,
ii) L est de la fluorescéine, et
iii) le squelette du tripode Y est choisi parmi les structures $Y_1$ à $Y_3$ suivantes :

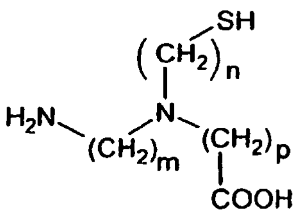
($Y_1$)

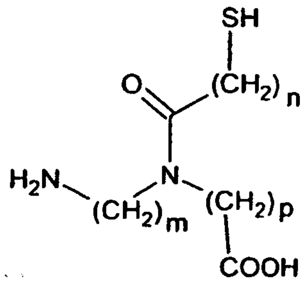
($Y_2$)

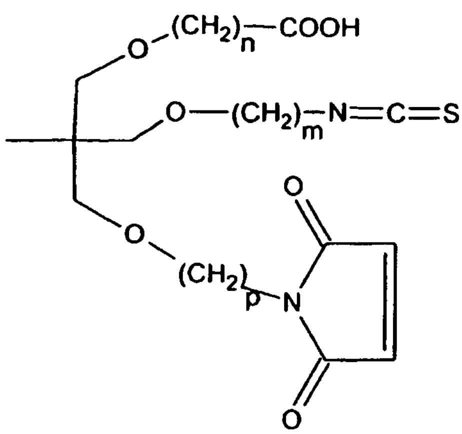
($Y_3$)

dans lesquelles n, m et p, identiques ou différents, sont des nombres entiers compris entre 1 et 20 inclusivement.

Parmi ces structures $Y_1$ à $Y_3$, les composés de formules ($Y'_1$) à ($Y'_3$) suivantes sont particulièrement préférés :

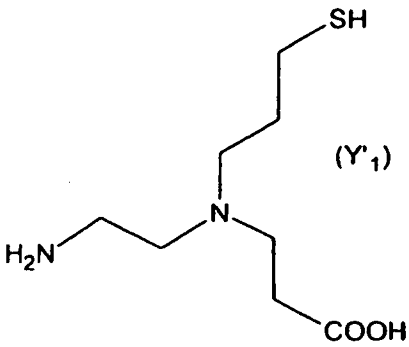

(Y'1)

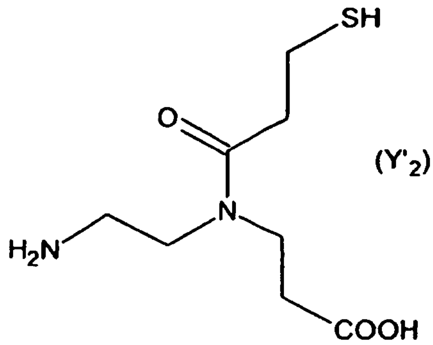

(Y'2)

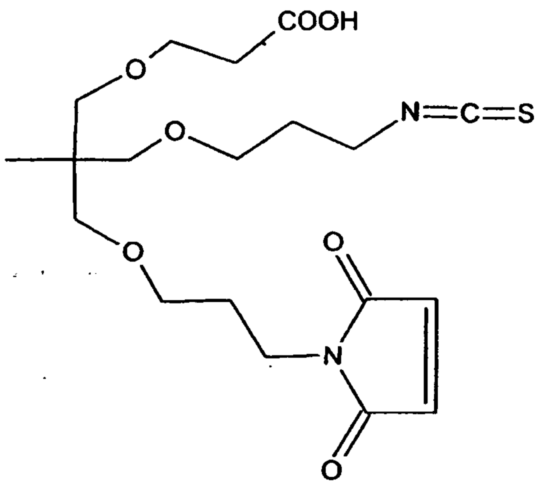

(Y'3)

Les tripodes Y utilisés dans le procédé conforme à l'Invention peuvent être préparés par analogie selon les procédés de synthèse organique et peptidique classiquement mis en oeuvre et bien connus de l'homme du métier.

Les complexes C de formules (I) tels que décrits précédemment sont des composés nouveaux en soi qui, à ce titre, constituent un autre objet de l'Invention.

Ces complexes C de formule (1) peuvent être préparés en complexant un tripode Y conforme à l'Invention avec un récepteur-Q selon les procédés classiquement mis en oeuvre dans l'état de la technique.

L'invention a également pour objet l'utilisation d'au moins un complexe C de formule (I) dans un procédé de détection en continu en phase hétérogène d'un analyte a au sein d'un échantillon fluide.

Enfin, l'Invention a pour objet un dispositif de détection en continu en phase hétérogène d'au moins un analyte a dans un échantillon fluide, ledit dispositif étant caractérisé par le fait qu'un échantillon fluide à analyser est intégré dans un milieu formant un flux s'écoulant sur au moins un support solide à la surface duquel est fixé au moins un tripode Y conforme à l'invention tel que décrit précédemment et spécifique de l'analyte a à détecter, un détecteur de luminescence disposé en regard du support solide est couplé à une commande de vanne asservie à un seuil d'intensité de signal émis par le détecteur et qui déclenche, pendant une durée déterminée, l'ouverture d'un réservoir renfermant un récepteur-Q apte à former un complexe avec le tripode Y, ce réservoir étant relié au support par une boucle de rétroaction qui débouche en amont du support solide sur lequel est fixé le tripode Y, afin de saturer et/ou régénérer ce dernier en récepteur-Q par passage dans le flux et complexation sur le tripode Y.

Selon des formes de réalisation particulières :

- les valeurs de l'intensité de la luminescence sont monitorées et accessoirement traduites en quantité d'analyte a par un système de calcul couplé au détecteur de luminescence ;
- un marqueur d'évènement tel que par exemple une alarme, est disposé dans la boucle de rétroaction afin de signaler une variation de l'intensité du signal supérieure à une valeur prédéterminée ;
- le support solide est un capillaire couplé à l'environnement contenant l'échantillon à analyser, le couplage étant réalisé soit par l'intermédiaire d'un ballon de capture où l'échantillon barbote dans un milieu correspondant à celui

du flux d'écoulement, soit par l'intermédiaire d'un conduit souple ;
- le flux est entraîné par la dépression produite par une pompe, un piston, ou équivalent.

Ces dispositifs peuvent notamment être utilisés pour détecter la présence d'un analyte a dans un milieu naturel tels que dans des lacs et rivières notamment ou dans des milieux industriels tels que piscines, usines, stations d'épuration, systèmes de ventilation ou de climatisation, etc. Ils peuvent, le cas échéant, être équipés d'un ballon de capture et d'un système de barbotage permettant de collecter des échantillons sous forme gazeuse tel que l'air et de solubiliser les constituants à détecter qu'ils renferment.

Outre les dispositions qui précèdent, l'Invention comprend encore d'autres dispositions qui ressortiront de la description qui va suivre, qui se réfère à des exemples de synthèse de squelettes de tripodes Y, à un exemple de synthèse d'un complexe de formule (1) et à deux exemples de détection de la substance P dans un échantillon fluide, ainsi qu'aux figures 1 à 8 annexées dans lesquelles :

- la figure 1 illustre une vue générale d'un exemple non limitatif de dispositif de détection en phase hétérogène selon l'Invention, pour mesurer la présence d'un analyte a dans une cuve de station d'épuration d'eau. Selon ce dispositif, un tuyau souple 1 relié à une sortie de cuve est monté sur un tube capillaire 3. Ce tube sert de support de fixation à deux types de tripode selon l'Invention, à savoir Ya et Yb, permettant la détection spécifique de deux types d'analytes. L'échantillon liquide provenant de la cuve est débité en flux continu (flèches Fa) dans le capillaire 3 par l'action d'une pompe péristaltique 5 puis évacué. A proximité du capillaire et face à chaque zone du capillaire sur laquelle sont fixés les tripodes Ya et Yb, il est prévu de positionner un, fluorimètre, 7a et 7b, détecteur de fluorescence. Chaque détecteur est relié à un réservoir de récepteurs-Q, 9a et 9b, respectivement spécifiques des tripodes Ya et Yb. Les réservoirs sont reliés par des canaux latéraux, 11a et 11b, et un canal commun 11c, au capillaire 3, en amont des zones sur lesquelles sont fixés les tripodes Ya et Yb par rapport au sens d'écoulement du flux. Le canal commun est soudé perpendiculairement au capillaire 3 et l'ensemble des canaux forme des boucles de rétroaction. Chaque détecteur est couplé à une commande de vanne, respectivement 13a et 13b, pour ouvrir le réservoir correspondant, respectivement 9a et 9b, et permettre à son contenu de se déverser dans le capillaire 3 (flèches Fb), puis de se complexer sur le tripode spécifique correspondant, Ya ou Yb, *via* les canaux 11a, 11b et 11c afin de régénérer ce tripode. Dans cet exemple, la régénération peut être réalisée en continu, le seuil de déclenchement de cette opération de régénération correspondant à la variation de luminescence minimale pouvant être détectée suivant la sensibilité du détecteur. Un marqueur d'événement 15a (respectivement 15b) est montée dans chaque boucle de rétroaction. En fonctionnement, et après étalonnage, l'intensité du signal fluorescent, 1a et 1b, émis par les tripodes Ya et Yb est mesurée par le fluorimètre, ce qui permet de calculer, à l'aide d'un calculateur annexé (non représenté), la concentration moyenne en analyte sous examen dans l'échantillon. La commande d'ouverture de vanne 13a (respectivement 13b) est asservie à l'intensité de signal fluorescent mesurée par le détecteur correspondant. L'ouverture de la vanne correspondante est alors déclenchée pendant la durée correspondant à la régénérescence des tripodes concernés. Si l'analyte a est présent dans l'échantillon lors de la rénégération, cela se traduit par une augmentation de la durée de celle-ci (une partie du récepteur-Q se complexant à l'analyte a). La durée de la régénération peut être déterminée par le temps nécessaire à un retour de la fluorescence au niveau basal ; si celle-ci est supérieure à une valeur prédéterminée, un marqueur d'évènement pourra être déclenché. Lorsque l'intensité du signal varie d'une quantité supérieure à une valeur prédéterminée, pendant un écart de temps donné, le marqueur d'évènement 15a (respectivement 15b) signale ce fait par un avertissement 16 visuel et/ou sonore. Le signal fluorescent correspondant à l'ensemble des analytes a ayant transité dans le capillaire entre deux régénérations, la lecture de la fluorescence peut également être effectuée à intervalles de temps réguliers. Dans d'autres exemples, lorsque l'échantillon est un gaz, le milieu mis en oeuvre et les moyens de création de flux sont adaptés par l'homme du métier.
- la figure 2 représente la fluorescence mesurée en unités arbitraires après immobilisation sur une plaque de microtitration, *via* la neutravidine, d'un tripode Y comprenant de la fluorescéine à titre de composé (L) et un analogue de la Substance P à titre de molécule (B), en fonction de la concentration en tripode en $\mu$M ;
- la figure 3 représente la courbe de diminution de la fluorescence (en unités arbitraires) d'un tripode comprenant de la fluorescéine à titre de composé (L) et un analogue de la Substance P à titre de molécule (B) en fonction de la quantité d'anticorps monoclonal anti-substance P marqué à la tetraméthyl rhodamine (mAb SP31-TMR) (en nM) qui se complexe au tripode ;
- la figure 4 représente le pourcentage d'inhibition par la substance P de la diminution de la fluorescence provoquée par le mAb SP31-TMR (% ID) est exprimé en fonction de la concentration en substance P (en nM) ;
- la figure 5 représente la fluorescence mesurée (en unités arbitraires) après mise en contact d'un support solide sur lequel est fixé un tripode Y conforme à l'Invention, ledit tripode étant ou non complexé au mAb SP31-TMR (puits Fl : fluorescence mesurée en absence de mAb SP31-TMR et de Substance P et puits $Fl_0$ : fluorescence mesurée en présence de mAb SP31-TMR mais en absence de Substance P), et avec différentes concentrations de substance

P (puits $Fl_x$ : fluorescence mesurée en présence de mAb SP31-TMR avec x = concentration en substance P : 1 ; 0,1 ou 0,01 μM), et ce lors d'un premier dosage, puis après régénération du support et enfin après une nouvelle mise en contact des puits avec les trois concentrations de substance P ;

- la figure 6 représente la moyenne de fluorescence mesurée (en unités arbitraires) pour chacun des puits Fl, $Fl_0$ et $Fl_x$, après 11 cycles de dosage de la substance P et de régénération ;
- la figure 7 représente la fluorescence (en unités arbitraires) mesurée pour chacun de 11 dosages effectués dans des puits Fl, $Fl_0$ et $Fl_x$, avec x = 1 μM de substance P ;
- la figure 8 représente l'évolution de la fluorescence (en unités arbitraires) en fonction du temps d'un capillaire fonctionnalisé par un tripode Y conforme à l'invention, mise en contact avec un anticorps monoclonal mAb SP31 marqué (Zone 1), puis avec de la substance P (Zone 2) et régénéré par mise en contact du tripode fixé au capillaire avec l'anticorps monoclonal mAb SP31 marqué (Zone 3.) ; les deux dernières étapes étant répétées trois fois.

**EXEMPLE 1 : PRÉPARATION D'UN SQUELETTE DE FORMULE ($Y'_1$) D'UN TRIPODE Y CONFORME À L'INVENTION**

On fait réagir un équivalent de 1,2-éthyldiamine avec deux équivalents de $(Boc)_2O$ pour conduire au bis-(tertiobutyloxycarbonylamino)-1,2-éthyl qui est ensuite mis à réagir avec un équivalent de phényl-3-bromopropanoate en présence d'hydrure de sodium pour conduire au composé de formule (Y'1a) suivante:

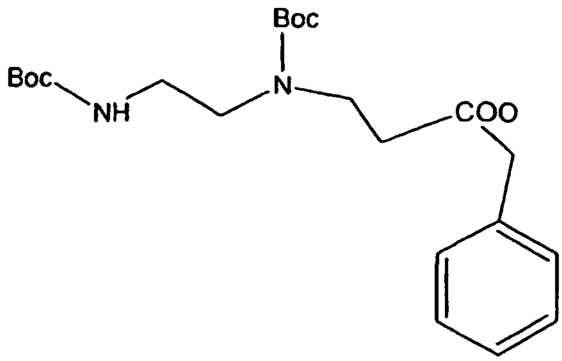

($Y'_1a$)

On déprotège le composé Y'la en milieu trifluoroacétique puis on réalise la condensation du composé déprotégé obtenu en présence de triéthylamine (TEA) et de un équivalent de chlorure de Z (avec Z = carbobenzyloxy) pour obtenir un composé de formule (Y'1b) suivante :

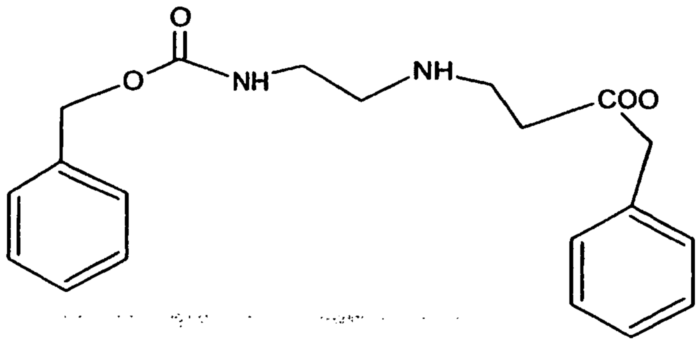

($Y'_1b$)

Le composé de formule (Y'1b) conduit ensuite, en présence de S-(3-chloropropyl) éthanethioate et de TEA, au composé de formule (Y'1c) suivante :

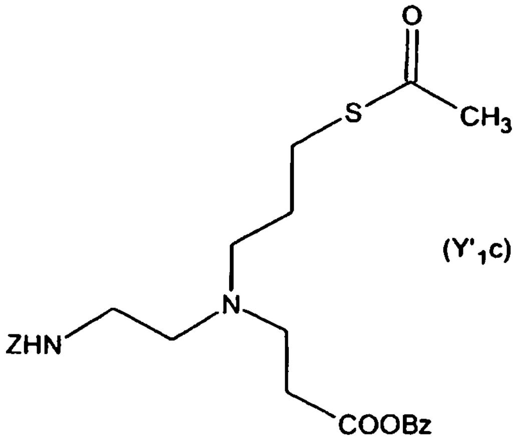

(Y'$_1$c)

dans laquelle Z a la même signification que celle indiquée pour le composé de formule (Y'1b) ci-dessus et Bz désigne un groupement benzyle.

On déprotège les fonctions acide et amine primaire du composé de formule (Y'1c) en présence de palladium sur charbon et la fonction thiol par action d'hydroxylamine pour conduire au composé de formule (Y'1).

## EXEMPLE 2 : PRÉPARATION D'UN SQUELETTE DE FORMULE (Y'$_2$) D'UN TRIPODE Y CONFORME À L'INVENTION

De l'acide 3-[(2-oxopropyl)thio]propanoïque, préparé à partir d'acide 3-bromopropanoïque et d'acide éthanethioïque est mis à réagir avec le composé de formule (Y'1b) obtenu ci-dessus à l'exemple 1 pour conduire au composé de formule (Y'2a) suivante :

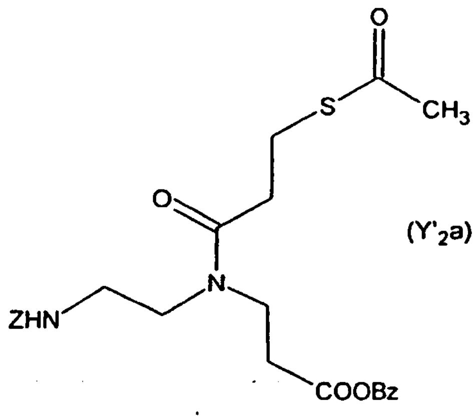

(Y'$_2$a)

dans laquelle Z désigne un groupement carbobenzyloxy et Bz désigne un groupement benzyle.

On déprotège les fonctions acide et amine primaire du composé de formule (Y'2a) ci-dessus en présence de palladium sur charbon et la fonction thiol par action d'hydroxylamine pour conduire au composé de formule (Y'2) décrit précédemment.

## EXEMPLE 3 : PRÉPARATION D'UN SQUELETTE DE FORMULE (Y'$_3$) D'UN TRIPODE Y CONFORME À L'INVENTION

On fait réagir un équivalent de 2-(hydroxyméthyl)-2-méthylpropane-1,3-diol et un équivalent d'éthyl 3-bromopropanoate en présence d'hydrure de sodium pour conduire à l'éthyl 3-[3-hydroxy-2-(hydroxyméthyl)-2-méthylpropoxy] propanoate. On fait ensuite réagir ce composé, en présence d'hydrure de sodium, avec un équivalent de *tert*-butyl 3-bromopropylcarbamate pour conduire au composé de formule (Y'3a) suivante :

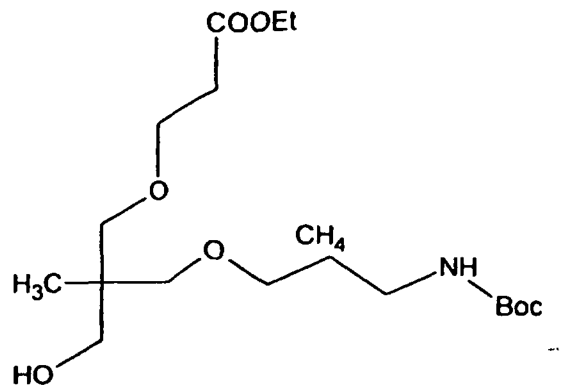

(Y'3a)

Le composé de formule (Y'3a) ci-dessus est ensuite déprotégé en milieu trifluoroacétique puis mis à réagir avec du dithioxométhane en présence de soude puis le groupement carboxylique est reprotégé par une mélange éthanol/acide sulfurique, pour conduire au composé de formule (Y'3b) suivante :

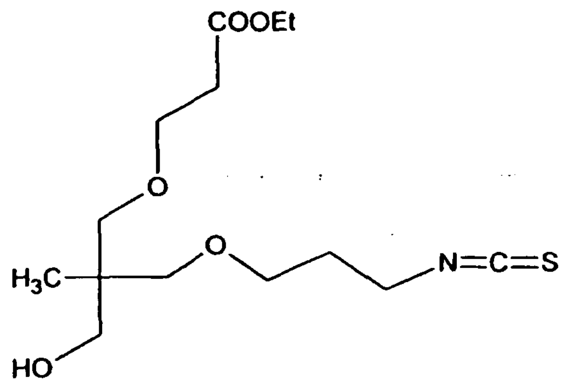

(Y'3b)

On fait ensuite réagir le composé de formule (Y'3b) ci-dessus avec du 1-(3-iodopropyl)-1H-pyrrole-2,5-dione en présence d'hydrure de sodium pour conduire au composé de formule (Y'3c) suivante :

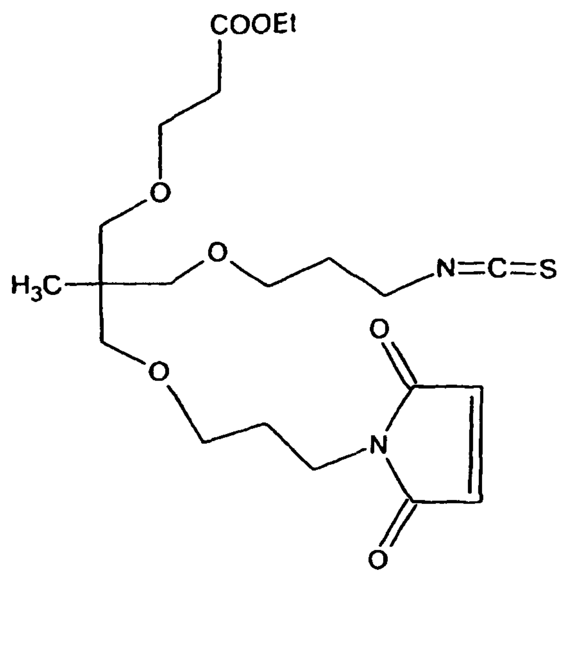

(Y'3c)

qui en présence de soude conduit au composé (Y'3).

## EXEMPLE 4 : PRÉPARATION D'UN TRIPODE Y CONFORME À L'INVENTION

Dans cet exemple, on illustre la préparation d'un tripode Y conforme à l'Invention comportant :

- un analogue de la substance P à titre de molécule (B),
- une fonction $NH_2$ permettant la fixation du tripode sur la surface d'un support solide ($F_3$),
- de la fluorescéine à titre de composé luminescent (L).

1) Préparation d'un analogue de la substance P

Pour mémoire, la séquence de la substance P (Poids moléculaire : PM = 1349) est la suivante :

Arg-Pro-Lys-Pro-Gln-Gln-Phe-Phe-Gly-Leu-Met-$CONH_2$

soit (SEQ ID N°1)-$CONH_2$.

On prépare un analogue de la substance P correspondant à la séquence suivante (PM : 1907) :

Lys-Ser-Ser-Lys(Biotine)-Arg-Pro-Ala-Pro-Gln-Gln-Phe-Phe-Gly-Ala-Met-$CONH_2$ ;

soit (SEQ ID N°2)-$CONH_2$

Pour ce faire, le couplage de l'analogue de la substance P et de la biotine ainsi que de la fonction $NH_2$ s'effectue au cours d'une synthèse peptidique selon une méthode connue de l'homme de l'art comme par exemple une synthèse en phase solide telle que décrite par Merrifield (J. Am. Chem. Soc., 1964, **85**, 2149-2154). L'analogue de la substance P est un peptide analogue de la substance P comportant une alanine en position 10 [$Ala^{10}$] qui a une réaction croisée de 13 % avec la substance P pour l'anticorps monoclonal SP31 (Déry O., Biopolymers, 1996, **39**, 67-74) qui est utilisé dans le dosage décrit ci-après comme récepteur. Cette plus faible affinité de l'anticorps monoclonal SP31 (mAb SP31) pour cet analogue par rapport à la substance P, permettra un meilleur déplacement de l'anticorps fixé sur la phase solide. Sur cet analogue, la lysine en position 3 (Lys3) a également été substituée par une alanine afin d'éliminer l'amine primaire présente sur la chaîne latérale. La lys3 n'étant pas dans le site de reconnaissance du mAb SP31, cette substitution ne modifie par l'affinité de l'anticorps pour l'analogue. Par ailleurs, l'extrémité N-terminale a été modifiée par ajout d'une lysine possédant une molécule de biotine sur la chaîne latérale (Fmoc-Lys(biotine)-OH), et deux Sérines suivies d'une Lysine possédant deux fonctions $NH_2$ dont une sur sa chaîne latérale.

Après la synthèse, le composé obtenu est purifié par chromatographie en phase liquide haute performance (HPLC) puis lyophilisé.

2) Marquage de l'analogue de la substance P avec de la fluorescéine (L)

100 $\mu$g du composé obtenu ci-dessus en 1) (5,28 $10^{-8}$ mol) sont dissous dans 100 $\mu$l de tampon borate 0,1 M de pH 9. On ajoute ensuite 30,8 $\mu$l (5,28 $10^{-7}$ mol) d'une solution de fluorescéine-NH-Succinimide (PM 586,55) (ester succinimidylique de l'acide hexanoïque (6-(fluorescéine-5-(et-6)-carboxamido) vendu sous la référence F-6129 par la société Molecular Probes, Inc.) à 10 mg/ml dans du diméthylformamide (DMF). Après 16 heures d'agitation à une température de 4°C, on ajoute 100 $\mu$l de tampon Tris-HCl 1 M pH 9 et on poursuit l'agitation pendant 16 heures à 4°C, afin d'inactiver les fonctions esters actives résiduelles. Enfin, on ajoute 769,2 $\mu$l de tampon phosphate de potassium 0,1M pH 7,4 et 0,01 % d'azide de sodium. On obtient une solution d'un tripode Y conforme à l'Invention à 52,8 $\mu$M). Ce tripode est conservé à une température de 4°C.

## EXEMPLE 5 : DÉTECTION ET DOSAGE DE LA SUBSTANCE P DANS UN ÉCHANTILLON

1) Préparation d'un récepteur-Q : marquage de l'anticorps monoclonal anti-substance P SP31 avec de la tetraméthyl rhodamine (TMR) (Q) (conjugué mAb SP31-TMR)

A 880 $\mu$l de mAb SP31 à 1,136 mg/ml (1 mg correspond à 6,67 nmole) en tampon phosphate 0,1 M pH 7,4, on ajoute 3,5 $\mu$l d'une solution de TMR (PM 527,5) (ester succinimidylique de la 5-(et-6)-carboxytetraméthylrhodamine ; vendu sous la référence C1171 par la société Molecular Probes, Inc., à 50 mg/ml dans le DMF (176 $\mu$g ; 333 nmole)). Après 16 heures d'agitation à 4°C, on ajoute 100 $\mu$l de tampon Tris-HCl 1M pH 9 et on poursuit l'agitation pendant 16 heures à 4°C, afin d'inactiver les fonctions esters actives résiduelles. Enfin, on sépare J'anticorps marqué de la TMR

libre par chromatographie d'exclusion sur gel G-25 (Sephadex G-25 fine, Amersham Biosciences) en tampon phosphate de potassium 0,1 M pH 7,4 renfermant 0,01 % d'azide de sodium. Les fractions correspondant à l'anticorps sont rassemblées et on mesure l'absorbance de la solution à 280 et à 555 nm à l'aide d'une cuve dont le trajet optique est de 1 cm.
La concentration en anticorps est calculée selon l'équation suivante :

$$\text{Concentration (M)} = [A_{280}-(A_{555} \times 0{,}3)] / \varepsilon$$

dans laquelle :

- $A_{280}$ est l'absorbance à 280 nm
- $A_{555}$ est l'absorbance à 555 nm
- 0,3 est le ratio de l'absorbance de la TMR à 280 nm et à 555 nm
- $\varepsilon$ est le coefficient d'extinction molaire (pour 1gG $\varepsilon$ = 203000 $cm^{-1}$ $M^{-1}$).

On obtient une concentration en anticorps marqué de 1,36 µM.
Le calcul du degré de marquage peut être fait selon l'équation suivante :

$$\text{Nombre de molécules de TMR par molécule d'anticorps} =$$

$$A_{555}/(65\,000 \times \text{concentration en anticorps})$$

dans laquelle le nombre 65 000 correspond au coefficient d'extinction molaire à 555 nm de la TMR.
On obtient 3,6 moles de TMR par mole d'anticorps.

2) Préparation du support solide : Fonctionnalisation de la surface par de la neutravidine

Afin de pouvoir immobiliser le tripode Y préparé ci-dessus à l'exemple 4 sur un support solide, ce dernier est, dans un premier temps, fonctionnalisé par de la neutravidine.

a) Réactifs utilisés

Tampon de saturation :

- tampon phosphate de potassium 0,1M pH 7,4 ;
- NaCl 0,15M ;
- Albumine sérique bovine (BSA) vendue sous la référence A-7906 par la société Sigma : 0,1 % ;
- Azide de sodium 0,01 %

Tampon de lavage :

- Tampon phosphate de potassium 0,01 M pH 7,4 ;
- Tween® 20 : 0,05 %

On dépose 100 µl d'une solution de neutravidine (vendue sous la référence 31 000 par la société Pierce) à 5 µg/ml dans du tampon phosphate de potassium 0,1 M pH 7,4 dans les puits de microplaques noires vendues sous la dénomination High Bind Matrix par la société VWR (référence 80120696). Après 16 heures à température ambiante, les plaques sont lavées avec du tampon de lavage puis saturées par dépôt de 300 µl/puits de tampon de saturation pendant 16 heures à température ambiante. Les plaques sont stockées avec le même tampon (300 µl/puits) à 4°C.

3) Fixation du tripode Y conforme à l'Invention (marqué à la fluorescéine) sur les plaques de microtitration

On prépare une gamme de dilution du tripode Y (marqué à la fluorescéine) synthétisé ci-dessus à l'exemple 4 en tampon phosphate de potassium 0,1 M pH 7,4 en réalisant un premier point de gamme à une concentration de 0,5 μM puis 4 dilutions successives au tiers (soit 0,167 μM ; 0,055 μM ; 0,0185 μM et 6,2.10$^{-3}$ μM). On dépose 100 μl de chaque concentration en double dans des puits revêtus de neutravidine ou pour lesquels on n'a effectué que l'étape de saturation (témoin négatif). Après une incubation sous agitation pendant 3 heures à température ambiante, on lave les plaques avec le tampon de lavage (5 fois) puis on dépose 100 μl de tampon phosphate de potassium 0,1 M pH 7,4. La lecture de la plaque est effectuée avec un fluorimètre Analyst AD system (LJL Biosystem) permettant la lecture de microplaques, à une longueur d'onde d'excitation de 485 nm et à une longueur d'onde d'émission de 530 nm qui sont les longueurs d'ondes d'excitation et d'émission correspondant à la fluorescéine.

Résultats

Les courbes obtenues avec neutravidine (carré noir) et sans neutravidine (triangle noir : témoin) sont représentées sur la figure 2 annexée sur laquelle la fluorescence (en unités arbitraires) est exprimée en fonction de la concentration en tripode Y conforme à l'Invention (μM).

Ces résultats montrent une augmentation de la fluorescence proportionnelle à celle de la concentration en tripode conforme à l'Invention jusqu'à une concentration de 0,17 μM puis un plateau correspondant à la saturation des sites de liaison de la neutravidine par le tripode. En absence de neutravidine immobilisée, aucune augmentation de la fluorescence n'est observée.

Dans les exemples qui suivent, le tripode Y de l'exemple sera utilisé à une concentration de 20 nm qui permet, au regard de ces résultats, d'obtenir un signal suffisant.

4) Saturation de la phase solide par le mAb SP31-TMR pour former un complexe C

On prépare une gamme de dilution du mAb SP31-TMR en tampon phosphate de potassium 0,1 M pH 7,4 en réalisant un premier point de gamme à une concentration de 100 nM puis 6 dilutions au tiers. On dépose 100 μl de chaque concentration en double avec 100 μl d'une solution du tripode Y de l'exemple 4 (solution à 20 nM dans du tampon phosphate de potassium 0,1 M pH 7,4) dans les puits d'une microplaque. Après incubation pendant 16 heures à 4°C, on lave les microplaques à l'aide du tampon de lavage (5 fois), puis on dépose 100 μl de tampon phosphate de potassium 0,1 M pH 7,4 et on mesure la fluorescence.

Les résultats obtenus sont représentés sur la Figure 3 annexées sur laquelle la fluorescence mesurée (en unités arbitraires) est exprimée en fonction de la concentration en mAb SP31-TMR (en nM).

Ces résultats montrent qu'à partir d'une concentration de 1 nM en anticorps, la fluorescence diminue proportionnellement à la concentration en mAb SP31-TMR, jusqu'à un maximum d'inhibition de 70 % obtenu à partir d'une concentration en mAb SP31-TMR de 33 nM. Ces résultats démontrent que :

i) l'anticorps se complexe au tripode Y conforme à l'Invention en reconnaissant l'épitope,
ii) la configuration du tripode Y conforme à l'invention permet un transfert d'énergie par résonance de la fluorescéine à la TMR, qui se traduit par une diminution de la fluorescence de la fluorescéine.

5) Détection de la substance P sur support solide

On prépare une gamme de dilution de Substance P en tampon phosphate de potassium 0,1 M pH 7,4 en réalisant un premier point de gamme à une concentration de 200 nM puis 6 dilutions au tiers. On dépose ensuite 100 μl du tripode préparé à l'exemple 4 (20 nM) et 100 μl de mAb SP31-TMR (30 nM) (ou 100 μl de tampon phosphate de potassium 0,1 M pH 7,4 pour les puits témoins négatifs) dans chaque puits. Après une incubation de 5 heures à température ambiante sous agitation, les plaques sont lavées à l'aide du tampon de lavage (5 fois) puis on dépose 100 μl de tampon phosphate de potassium 0,1 M pH 7,4 et on mesure la fluorescence.

Les résultats obtenus sont représentés sur la figure 4 annexée sur laquelle le pourcentage d'inhibition par la Substance P de la diminution de la fluorescence provoquée par le mAb SP31-TMR (% ID) est exprimé en fonction de la concentration en substance P (en nM).

Ce pourcentage d'inhibition est calculé à l'aide de l'équation suivante :

$$\% ID = [(Fl_x - Fl_0)/(Fl\text{-}Fl_0)]x100$$

dans laquelle :

- $Fl_x$ correspond à la fluorescence mesurée en présence de Substance P à la concentration x ;
- Fl correspond à la fluorescence mesurée en absence de mAb SP31-TMR et de Substance P ;
- $Fl_0$ correspond à la fluorescence mesurée en présence de mAb SP31-TMR et absence de Substance P.

Ces résultats montrent, comme attendu, que l'augmentation de la concentration en Substance P induit une augmentation de la fluorescence (correspondant à l'inhibition de la diminution) de la fluorescéine. On obtient ainsi une courbe standard dont la limite de détection en Substance P est d'environ 2 nM.

6) Test de régénération de la phase solide

On prépare trois dilutions de Substance P (respectivement 1 ; 0,1 et 0,01 μM) en tampon phosphate de potassium 0,1 M pH 7,4. On dépose ensuite 100 μl du tripode Y tel que préparé ci-dessus à l'exemple 4 (20 nM) et 100 μl de mAb SP31-TMR (50 nM) (ou 100 μl de tampon phosphate de potassium 0,1 M pH 7,4 pour les puits témoins négatifs : puits $Fl_0$) dans chaque puits. On utilise le mAb SP31-TMR à 50 nM afin de complexer tous les tripodes présents sur la phase solide. Après une incubation de 16 heures à 4°C, on lave les plaques à l'aide du tampon de lavage (5 fois) puis on dépose 100 μl de chaque concentration de Substance P en triple (puits $Fl_x$). Les puits Fl et $Fl_0$ sont également réalisés en triple. Après une incubation de 5 heures à température ambiante sous agitation, on lave les plaques à l'aide du tampon de lavage (5 fois) puis on dépose 100 μl de tapon phosphate de potassium 0,1 M pH 7,4 et on mesure la fluorescence (premier dosage).

On lave à nouveau les plaques de la même façon que précédemment et on incube 100 μl de mAb SP31-TMR (50 nM) (ou 100 μl de tampon phosphate de potassium 0,1 M pH 7,4 pour les puits Fl) pendant 16 heures à 4°C. Les plaques sont à nouveau lavées (5 fois), puis on dépose 100 μl de tampon phosphate de potassium 0,1 M pH 7,4 et on mesure la fluorescence (étape de régénération des plaques).

On lave de nouveau les plaques (5 fois) et on dépose soit 100 μl de tampon phosphate de potassium pour les puits Fl et $Fl_0$ soit 100 μl de Substance P (à 1 ; 0,1 ou 0,01 μM). Après une incubation de 5 heures à température ambiante sous agitation, on lave les plaques (5 fois), puis on dépose 100 μl de tampon phosphate de potassium 0,1 M pH 7,4 et on mesure la fluorescence (deuxième dosage).

Les résultats obtenus sont représentés sur la figure 5 annexée sur laquelle la fluorescence (en unités arbitraires) mesurée pour les puits Fl, $Fl_0$ et $Fl_x$ est exprimée pour les premier et deuxième dosages ainsi que pour la mesure faite après l'étape de régénération.

Ces résultats mettent en évidence que :

i) les différentes étapes du procédé de détection (dépôts, lavages), n'entraînent pas la dissociation du tripode Y conforme à l'Invention de la phase solide : la fluorescence mesurée dans les puits Fl est constante ;
ii) le dépôt du mAb SP31 permet une régénération homogène de la phase solide : mêmes valeurs de fluorescence pour tous les puits $Fl_x$ et $Fl_0$ ;
iii) la régénération est totale : la fluorescence mesurée pour les puits $Fl_x$ après l'étape de régénération est similaire à celle des puits $Fl_0$ effectuée au premier dosage ;
iv) cette étape de régénération est compatible avec un deuxième dosage effectué ultérieurement sur la même phase solide car on obtient les mêmes valeurs de fluorescence pour des concentrations en Substance P identiques.

7) Mise en évidence de la faisabilité de dosages répétés sur une même phase solide

On prépare trois dilutions de Substance P (1 ; 0,1 et 0,01 μM) en tampon phosphate de potassium 0,1 m pH 7,4.

i) On dépose 100 μl du tripode préparé ci-dessus à l'exemple 4 (20 nM) et 100 μl de mAb SP31-TMR (50 nM) ou 100 μl de tampon phosphate de potassium 0,1 M pH 7,4 (témoin négatif : puits $Fl_0$) dans chaque puits d'une microplaque. On utilise le mAb SP31-TMR à la concentration de 50 nM afin de complexer toue les tripodes présents sur la phase solide. Après une incubation de 16 heures à 4°C, on lave la microplaque à l'aide du tampon de lavage (5 fois) puis on dépose 100 μl de chaque concentration de Substance P en triple. Les puits Fl et $Fl_0$ sont également réalisés en triple. Après une incubation de 5 heures à température ambiante sous agitation, on lave la microplaque (5 fois) puis on dépose 100 μl de tampon phosphate de potassium 0,1 M pH 7,4 et on mesure la fluorescence (premier dosage).
ii) On lave à nouveau la plaque (5 fois) et on incube 100 μl de mAb SP31-TMR (50 nM) (ou 100 μl de tampon phosphate de potassium 0,1M pH 7,4 pour les puits Fl) pendant 16 heures à 4°C (étape de régénération).
iii) On lave à nouveau la plaque (5 fois) et on dépose soit 100 μl de tampon phosphate pour Fl et $Fl_0$, soit 100 μl

de Substance P (à 1 ; 0,1 ou 0,01 μM). Après une incubation de 5 heures à température ambiante sous agitation, on lave la plaque (5 fois), puis on dépose 100 μl de tampon phosphate et on mesure la fluorescence (deuxième dosage).

Pour les dosages suivants, on répète les étapes ii) et iii) en faisant varier les puits dans lesquels sont déposés le tampon phosphate pour $FI_0$ et les différentes concentrations de Substance P.

On réalise ainsi 11 dosages successifs sur la même phase solide.

Les résultats obtenus sont représentés sur les figures 6 et 7 annexées.

Sur la figure 6, la fluorescence (en unités arbitraires) correspond, pour chaque puits FI, $FI_0$ et $FI_x$, à la moyenne des 11 dosages effectués. Sur la figure 7, la fluorescence (en unités arbitraires) correspond aux valeurs obtenues pour chacun des 11 dosages effectués pour les puits FI, $FI_0$ et $FI_x$ avec x = 1 μM de Substance P.

Ces résultats montrent que pour chaque concentration de Substance P, les valeurs de fluorescence sont homogènes et supérieures à celles obtenues en absence de Substance P. De plus, les fluctuations observées sont aléatoires ; il n'y a pas de baisse ou d'augmentation progressive de la fluorescence au fil des dosages.

Ces résultats montrent également que le procédé de détection conforme à la présente Invention permet le dosage répété d'un analyte sur une phase solide, chacun des puits (ou site de réaction) pouvant être régénéré de façon équivalente et permettre une nouvelle détection ultérieure.

## EXEMPLE 6 : DETECTION DE LA SUBSTANCE P ET REGENERATION EN CAPILLAIRE

### 1) Mode opératoire

a) Préparation des réactifs utilisés

- Marquage du tripode avec une molécule fluorescente :
  Le marquage de l'analogue de la substance P de séquence SEQ ID N°2 (tel décrit ci-dessus à l'exemple 4) avec une molécule fluorescente vendue sous la dénomination commerciale Alexa Fluor® 532 Protein Labeling Kit, référence A-10236 (Molecular Probes) est effectué selon le protocole décrit ci-dessus à l'exemple 4. On obtient un tripode-Alexa 532
- Préparation d'un récepteur-Q : marquage de l'anticorps mAb SP 31 avec une molécule fluorescente (conjugué mAb SP31-Alexa 647) :
  Le marquage du mAb SP31 avec une molécule fluorescente vendue sous la dénomination commerciale Alexa® Fluor 647 Succinimidyl Esters, référence A-20006 (Molecular Probes) est effectué selon le protocole décrit ci-dessus à l'exemple 5, étape 1). On obtient un anticorps marqué mAb SP31-Alexa 647.
- Préparation des capillaires :
  On utilise des capillaires en borosilicate de section carrée (arête intérieure = 0,5 mm, extérieur = 1 mm, longueur = 25 mm) (Wale Apparatus). Les capillaires sont fonctionnalisés avec de la neutravidine, puis incubés dans une solution de tripode-Alexa 532 (solutions à 5μM) durant 1 nuit à 4°C.

b) Protocole expérimental

Le capillaire fonctionnalisé est connecté par des tubulures Manifold de diamètre intérieur 0,51 mm (Bioblock) à une pompe Gilson, permettant le passage successif de différentes solutions.

Pour la mesure de la fluorescence, on utilise un microscope à fluorescence (excitation par une lampe à vapeur de mercure) équipé d'une caméra CCD refroidie HisIs 23 (Europixel, Italie) et d'un "shutter", pilotés par ordinateur.

L'acquisition des images est réalisée sur la même zone du capillaire tout au long de l'expérience, avec un temps d'exposition de 50 msec en employant un filtre à l'excitation à 530-560 nm et un filtre à l'émission à 575-645 nm.

Après lavage du capillaire avec du tampon de lavage tel que décrit ci-dessus à l'exemple 5, le capillaire fonctionnalisé est rempli de tampon phosphate 0,1 M pH 8,5 et la fluorescence initiale à t=0 est lue.

Une solution de mAb SP31-Alexa 647 à 2,62 μM est ensuite introduite dans le capillaire. Après 10 minutes le capillaire est lavé (tampon de lavage durant 2 minutes à un débit de 35 μl/min) puis rempli de tampon phosphate 0,1 M pH 8,5 et la mesure de la fluorescence après "quenching" est effectuée.

On introduit par la suite une solution de Substance P à 10 nmol/ml pendant 80 minutes. Après une première mesure à t=15 secondes, les mesures sont réalisées toutes les 5 minutes.

Après lavage du capillaire (tampon de lavage puis tampon phosphate 0,1 M pH 8,5 pendant 2 minutes pour chacun à 35 μl/min), la phase solide est régénérée par une nouvelle incubation de mAb SP31-Alexa 647. L'expérience

est ainsi répétée 3 fois.

**2) Résultats**

Les résultats obtenus lors de cette expérience sont représentés sur la figure 8 annexée sur laquelle la fluorescence (en unités arbitraires) est exprimée en fonction du temps (minutes).

Ces résultats montrent que l'extinction ("quench") de la fluorescence du tripode-Alexa 532 par le mAb SP 31-Alexa 647 (zone 1 de la courbe), l'augmentation de la fluorescence en présence de substance P. (zone 2 de la courbe) et l'étape de régénération par l'introduction de mAb SP 31-Alexa 647 (zone 3 de la courbe) restent effectifs sur support solide tel que les capillaires et avec d'autres groupements luminescents (L) et accepteurs (Q) que ceux utilisés dans l'exemple 5.

## Revendications

1. Procédé de détection d'un analyte a dans un échantillon fluide, **caractérisé par le fait qu**'il comprend les étapes suivantes :

    1) la saturation d'un support solide comportant, sur au moins une partie de sa surface, au moins un réactif trifonctionnel (tripode Y) comportant les trois pôles fonctionnels suivant :

        i) un groupement luminescent (L),
        ii) une molécule (B) choisie parmi l'analyte a, un analogue de l'analyte a ou un fragment de l'analyte a; et
        iii) une fonction assurant la fixation dudit réactif trifonctionnel sur la surface dudit support solide ;

    par un récepteur de l'analyte a, ledit récepteur étant marqué par un composé accepteur (Q) (récepteur-Q) de la luminescence du groupement L, pour former un complexe C entre ladite molécule (B) et ledit récepteur-Q ;
    2) la mise en contact du support solide obtenu à l'étape 1) avec un échantillon fluide susceptible de renfermer l'analyte a à détecter ;
    3) la mesure de l'intensité du signal émis par le groupement L qui est proportionnelle à la quantité d'analyte a présent au sein de l'échantillon fluide ; et
    4) la régénération du support solide par mise en contact dudit support solide avec du récepteur-Q.

2. Procédé selon la revendication 1, **caractérisé par le fait que** plusieurs types de tripodes Y, différant les uns des autres par la nature de la molécule (B) qu'ils comprennent, sont fixés sur des zones distinctes et connues du support solide.

3. Procédé selon la revendication 1 ou 2, **caractérisé par le fait que** les étapes 3) et 4) sont réalisées en continu.

4. Procédé selon l'une quelconque des revendications précédentes, **caractérisé par le fait que** le support solide est choisi parmi les verres, les plastiques, les céramiques, les métaux et les métalloïdes.

5. Procédé selon l'une quelconque des revendications précédentes, **caractérisé par le fait que** le support se présente sous la forme de tube, de capillaire, de plaque ou de bille.

6. Procédé selon l'une quelconque des revendications précédentes, **caractérisé par le fait que** l'échantillon fluide est constitué par de l'eau, un milieu biologique liquide ou un milieu liquide contenant des molécules gazeuses dissoutes ou provenant d'échantillons solides.

7. Procédé selon l'une quelconque des revendications précédentes, **caractérisé par le fait que** la mesure de l'intensité du signal émis lors de l'étape 3) est effectuée par un détecteur de luminescence.

8. Procédé selon l'une quelconque des revendications précédentes, **caractérisé par le fait que** le complexe C formé à l'issue de l'étape 1) de saturation est choisi parmi les complexes de formule (I) suivante :

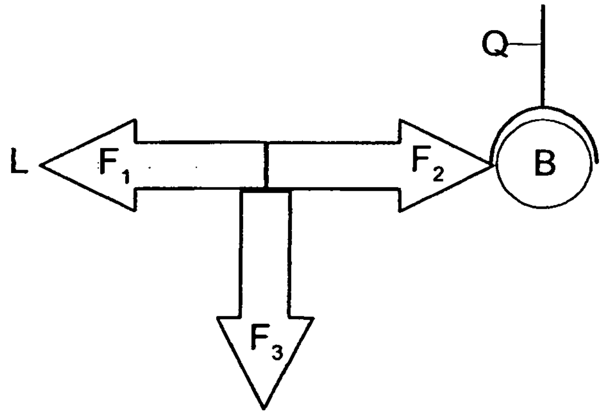

(I)

dans laquelle :

- les flèches représentent la structure du squelette du tripode Y qui est un bras de liaison constitué par une chaîne peptidique, nucléotidique, glucosidique ou par une chaîne hydrocarbonée linéaire ou ramifiée, saturée ou insaturée ; lesdites chaînes étant éventuellement substituées, interrompues et/ou terminées par un ou plusieurs hétéroatomes tels que N, O ou S et/ou par un ou plusieurs acides aminés, et comportant trois fonctions chimiques réactives $F_1$, $F_2$ et $F_3$ ;
- L représente un groupement luminescent lié de façon covalente au tripode Y par l'intermédiaire de la fonction chimique réactive $F_1$;
- B représente un analyte a, un analogue structurel d'un analyte a ou un fragment d'un analyte a sur lequel est fixé, de façon non covalente et réversible, un récepteur spécifique de l'analyte a, ledit récepteur étant marqué par un composé Q ; la molécule (B) étant liée de façon covalente au tripode Y par l'intermédiaire de la fonction chimique réactive $F_2$;
- Q représente un composé accepteur de la luminescence du groupement L;
- $F_3$ représente une fonction chimique réactive apte à permettre la fixation du tripode Y sur la surface du support solide.

9. Procédé selon la revendication 8, **caractérisé par le fait que** les fonctions $F_1$, $F_2$, $F_3$, indépendamment l'une de l'autre, assurent :

i) soit une liaison directe *via* une fonction chimique correspondante présente sur le composé luminescent, la molécule (B) ou la phase solide ;
ii) soit une liaison indirecte, et dans ce cas, la liaison est réalisée en couplant à l'une au moins des fonctions $F_1$, $F_2$ et/ou $F_3$, une molécule $M_1$ apte à former un complexe avec une molécule $M_2$ préalablement fixée sur au moins une partie de la surface de la phase solide, sur la molécule (B) et/ou sur le groupement luminescent.

10. Procédé selon la revendication 8 ou 9, **caractérisé par le fait que** les fonctions $F_1$, $F_2$ et $F_3$, identiques ou différentes, sont choisies parmi les fonctions thiols ; amines ; alcools ; acides ; esters ; isothiocyanates ; isocyanates ; acylazides ; chlorures de sulfonyle ; aldéhydes ; glyoxals ; époxydes ; oxiranes ; carbonates ; imidoesters ; carbodiimides ; maléimides ; nitriles, aziridines ; acryloyl ; les dérivés halogénés ; les groupements disulfides ; phosphorées ; diazo ; carbonyldiimidazole ; hydrazides ; arylazides ; hydrazines ; diazirines ; magnésiens ; lithiens ; cuprates ; zinciques et les systèmes insaturés.

11. Procédé selon la revendication 10, **caractérisé par le fait que** les fonctions $F_1$, $F_2$ et $F_3$ sont choisies parmi les fonctions amines de formule $R\text{-}NH_2$, R-NH-, $(R)_3\text{-}N$, R-NH-OR et $NH_2\text{-}OR$ ; les fonctions alcool R-OH ; et les groupements halogénés de formule R-X avec X représentant un atome d'halogène ; étant entendu que dans lesdites formules R représente un radical alkyle, aryle, vinyle, ou allyle.

12. Procédé selon l'une quelconque des revendications précédentes, **caractérisé par le fait que** les groupements luminescents sont choisis parmi la fluorescéine (fluorescéinate de sodium) et ses dérivés ; la rhodamine et ses dérivés ; le diaminidophényl indo (DAPI) ; l'acridine ; les colorants fluorescents à amines réactives ; les colorants fluorescents vendus sous les dénominations commerciales BODIPY® ; les colorants Cascade Blue, Cy2, Cy3,

Cy3.5, Cy5, Cy5.5, Cy7, DABCYL® et EDANS® ; l'Eosine ; l'Erythrosine, le 6-Fam et le Texas Red.

**13.** Procédé selon l'une quelconque des revendications précédentes, **caractérisé par le fait que** les récepteurs sont choisis parmi les anticorps sous forme entière, fragmentaire ou recombinante, les récepteurs biologiques, les acides nucléiques, les acides peptidonucléiques, les lectine, les protéines transporteuses, les chélates et les récepteurs synthétiques.

**14.** Procédé selon l'une quelconque des revendications précédentes, **caractérisé par le fait que** ledit récepteur présente une plus grande affinité pour l'analyte a que pour la molécule (B).

**15.** Procédé selon l'une quelconque des revendications précédentes, **caractérisé par le fait que** le composé accepteur (Q) est choisi parmi la rhodamine et ses dérivés, les composés fluorescents cités à la revendication 12, des molécules non-fluorescentes choisies parmi les composés vendus sous les dénominations commerciales Black Hole Quencher® 1, 2 et 3, Nanogold Particules®, Eclipse Dark Quencher®, Elle Quencher®, le vert de malachite, et les colorants QSY® 7, QSY® 9 et QSY® 21.

**16.** Procédé selon l'une quelconque des revendications 8 à 15, **caractérisé par le fait que** les complexes de formule (1) sont choisis parmi les composés dans lesquels :

i) (B) est choisi parmi les peptides, les protéines, les oligonucléolides, les sucres et les acides peptidonucléiques,
ii) L est de la fluorescéine, et
iii) le squelette du tripode Y est choisi parmi les structures $Y_1$ à $Y_3$ suivantes:

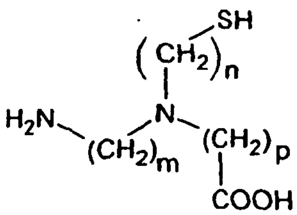

$(Y_1)$

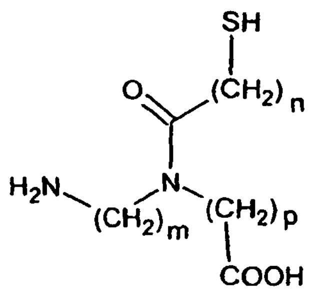

$(Y_2)$

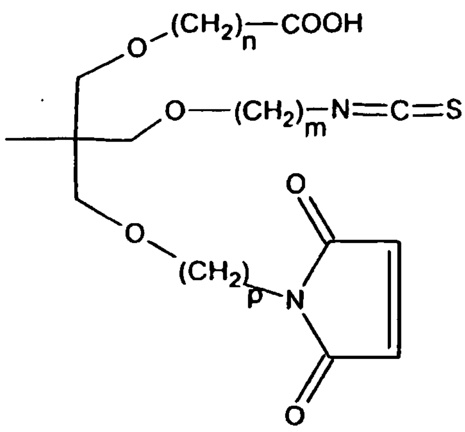

$(Y_3)$

dans lesquelles n, m et p, identiques ou différents, sont des nombres entiers compris entre 1 et 20 inclusivement.

**17.** Procédé selon la revendication 16, **caractérisé par le fait que** les structures $Y_1$ à $Y_3$ sont choisies parmi les composés de formules $(Y'_1)$ à $(Y'_3)$ suivantes :

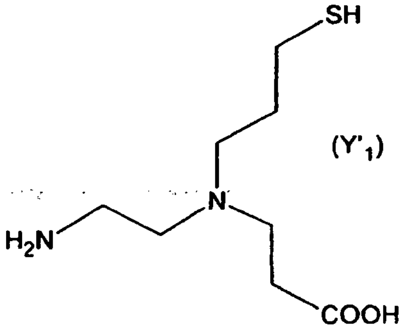

(Y'₁)

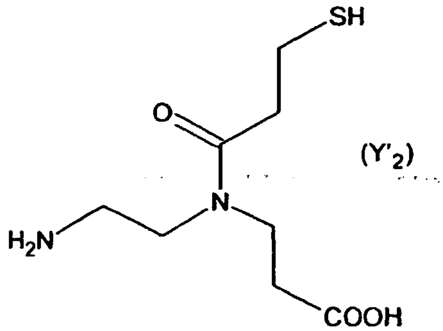

(Y'₂)

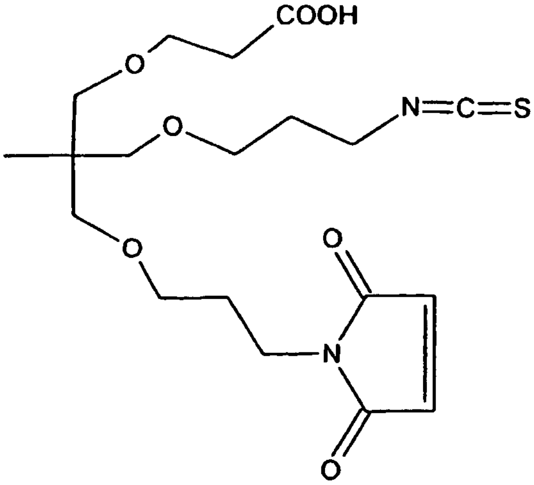

(Y'₃)

**18.** Complexes C **caractérisés par le fait qu**'ils répondent à la formule (I) suivante :

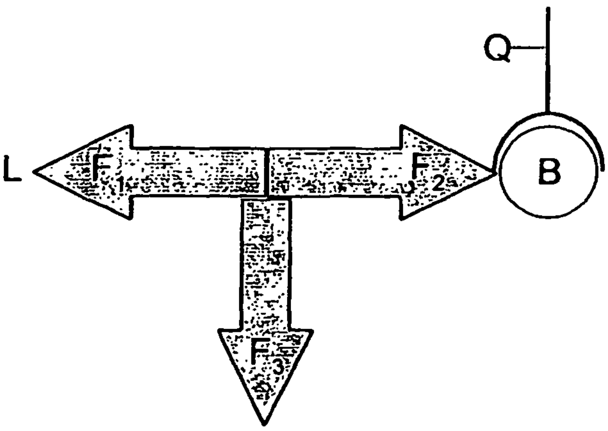


(I)

dans laquelle L, B, Q, les flèches et $F_1$, $F_2$ et $F_3$ sont tels que définis à l'une quelconque des revendications 8 à 17.

**19.** Utilisation d'au moins un complexe C de formule (I) tel que défini à l'une quelconque des revendications 8 à 16, dans un procédé de détection en continu en phase hétérogène d'un analyte a̲ au sein d'un échantillon fluide.

**20.** Dispositif de détection en continu en phase hétérogène d'au moins un analyte a̲ dans un échantillon fluide, ledit dispositif étant **caractérisé par le fait qu**'un échantillon fluide à analyser est intégré dans un milieu formant un flux

s'écoulant sur au moins un support solide à la surface duquel est fixé au moins un tripode Y tel que défini à la revendication 1 et spécifique de l'analyte a à détecter, un détecteur de luminescence disposé en regard du support solide est couplé à une commande de vanne asservie à un seuil d'intensité de signal émis par le détecteur et qui déclenche, pendant une durée déterminée, l'ouverture d'un réservoir renfermant un récepteur-Q apte à former un complexe avec le tripode Y, ce réservoir étant relié au support par une boucle de rétroaction qui débouche en amont du support solide sur lequel est fixé le tripode Y, afin de saturer et/ou régénérer ce dernier en récepteur-Q par passage dans le flux et complexation sur le tripode Y.

**21.** Dispositif selon la revendication 20, dans lequel les valeurs de l'intensité de la luminescence sont monitorées et accessoirement traduites en quantité d'analyte a par un système de calcul couplé au détecteur de luminescence.

**22.** Dispositif selon la revendication 20 ou 21, dans lequel un marqueur d'évènement est disposé dans la boucle de rétroaction afin de signaler une variation de l'intensité du signal supérieure à une valeur prédéterminée.

**23.** Dispositif selon l'une quelconque des revendications 20 à 22, dans lequel le support solide est un capillaire couplé à l'environnement contenant l'échantillon à analyser, le couplage étant réalisé soit par l'intermédiaire d'un ballon de capture où l'échantillon barbote dans un milieu correspondant à celui du flux d'écoulement, soit par l'intermédiaire d'un conduit souple.

**24.** Dispositif selon l'une quelconque des revendications 20 à 23, dans lequel le flux est entraîné par la dépression produite par une pompe, un piston, ou équivalent.

**25.** Dispositif selon l'une quelconque des revendications 20 à 24, **caractérisé par le fait qu**'il est équipé d'un ballon de capture et d'un système de barbotage permettant de collecter des échantillons sous forme gazeuse et de solubiliser les constituants à détecter qu'ils renferment.

**26.** Utilisation d'un dispositif tel que défini à l'une quelconque des revendications 20 à 25, pour détecter la présence d'un analyte a dans un milieu naturel ou industriel.

**27.** Utilisation selon la revendication 26, **caractérisé par le fait que** le dispositif est utilisé en lacs, rivières, piscines, usines, stations d'épuration, systèmes de ventilation ou de climatisation.

## Claims

**1.** Method of detecting an analyte a in a liquid sample, **characterised in that** it comprises the following steps:

1) saturating a solid support having, on at least part of its surface, at least one trifunctional reagent (tripod Y) having the following three functional poles:

i) a luminescent group (L),
ii) a molecule (B) selected from the analyte a, an analogue of the analyte a and a fragment of the analyte a; and
iii) a function which serves to fix said trifunctional reagent to the surface of said solid support;

with a receptor for the analyte a, said receptor being labelled with an acceptor compound (Q) (receptor-Q) for the luminescence of the group L, in order to form a complex C between said molecule (B) and said receptor-Q;
2) bringing the solid support obtained in step 1) into contact with a liquid sample which might contain the analyte a to be detected;
3) measuring the intensity of the signal emitted by the group L, which is proportional to the quantity of analyte a present in the liquid sample; and
4) regenerating the solid support by bringing said solid support into contact with receptor-Q.

**2.** Method according to claim 1, **characterised in that** several types of tripod Y, which differ from one another by the nature of the molecule (B) that they comprise, are fixed to discrete and known areas of the solid support.

**3.** Method according to claim 1 or 2, **characterised in that** steps 3) and 4) are carried out continuously.

**4.** Method according to any one of the preceding claims, **characterised in that** the solid support is selected from

glasses, plastics materials, ceramics materials, metals and metalloids.

5. Method according to any one of the preceding claims, **characterised in that** the support is in the form of a tube, a capillary, a plate or a spherule.

6. Method according to any one of the preceding claims, **characterised in that** the liquid sample is constituted by water, a liquid biological medium or a liquid medium containing dissolved gas molecules or molecules originating from solid samples.

7. Method according to any one of the preceding claims, **characterised in that** measurement of the intensity of the signal emitted in step 3) is effected by a luminescence detector.

8. Method according to any one of the preceding claims, **characterised in that** the complex C formed at the end of the saturation step 1) is selected from the complexes of the following formula (I):

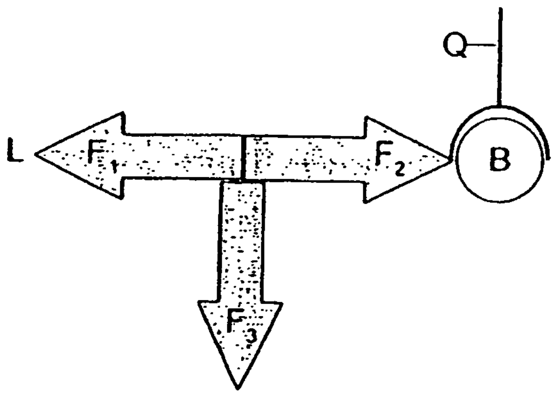


**(I)**

in which:

- the arrows represent the structure of the skeleton of the tripod Y, which is a linking arm constituted by a peptide, nucleotide or glucoside chain or by a linear or branched, saturated or unsaturated hydrocarbon chain; said chains being optionally substituted, interrupted and/or terminated by one or more hetero atoms such as N, O or S and/or by one or more amino acids, and having three reactive chemical functions $F_1$, $F_2$ and $F_3$;
- L represents a luminescent group covalently bonded to the tripod Y by way of the reactive chemical function $F_1$;
- B represents an analyte a, a structural analogue of an analyte a or a fragment of an analyte a to which there is fixed, in a non-covalent and reversible manner, a specific receptor for the analyte a, said receptor being labelled by a compound Q; the molecule B being covalently bonded to the tripod Y by way of the reactive chemical function $F_2$;
- Q represents an acceptor compound for the luminescence of the group L;
- $F_3$ represents a reactive chemical function capable of permitting the fixing of the tripod Y to the surface of the solid support.

9. Method according to claim 8, **characterised in that** the functions $F_1$, $F_2$, $F_3$, independently of one another, provide:

i) either a direct bond via a corresponding chemical function present on the luminescent compound, the molecule (B) or the solid phase;
ii) or an indirect bond, in which case the bond is produced by coupling to at least one of the functions $F_1$, $F_2$ and/or $F_3$ a molecule $M_1$ capable of forming a complex with a molecule $M_2$ which has previously been fixed to at least part of the surface of the solid phase, to the molecule (B) and/or to the luminescent group.

10. Method according to claim 8 or 9, **characterised in that** the functions $F_1$, $F_2$ and $F_3$, which may be identical or different, are selected from the functions thiols; amines; alcohols; acids; esters; isothiocyanates; isocyanates; acyl azides; sulfonyl chlorides; aldehydes; glyoxals; epoxides; oxiranes; carbonates; imidoesters; carbodiimides; maleimides; nitriles; aziridines; acryloyl; halogenated derivatives; disulfide groups; phosphorus-containing functions; diazo; carbonyldiimidazole; hydrazides; aryl azides; hydrazines; diazirines; magnesium-containing functions; lithium-containing functions; cuprates; zinc-containing functions and unsaturated systems.

**11.** Method according to claim 10, **characterised in that** the functions $F_1$, $F_2$ and $F_3$ are selected from amine functions of formula $R-NH_2$, R-NH-, $(R)_3-N$, R-NH-OR and $NH_2-OR$; alcohol functions R-OH; and halogenated groups of the formula R-X wherein X represents a halogen atom; it being understood that, in said formulae, R represents an alkyl, aryl, vinyl or allyl radical.

**12.** Method according to any one of the preceding claims, **characterised in that** the luminescent groups are selected from fluorescein (sodium fluoresceinate) and its derivatives; rhodamine and its derivatives; diamidinophenylindole (DAPI); acridine; reactive-amine-containing fluorescent dyes; fluorescent dyes sold under the commercial names BODIPY®; the dyes Cascade Blue, Cy2, Cy3, Cy3.5, Cy5, Cy5.5, Cy7, DABCYL® and EDANS®; Eosine; Erythrosine, 6-Fam and Texas Red.

**13.** Method according to any one of the preceding claims, **characterised in that** the receptors are selected from antibodies in whole, fragmentary or recombinant form, biological receptors, nucleic acids, peptidonucleic acids, lectins, transport proteins, chelates and synthetic receptors.

**14.** Method according to any one of the preceding claims, **characterised in that** said receptor has a greater affinity for the analyte a than for the molecule (B).

**15.** Method according to any one of the preceding claims, **characterised in that** the acceptor compound (Q) is selected from rhodamine and its derivatives, the fluorescent compounds mentioned in claim 12, non-fluorescent molecules selected from the compounds sold under the commercial names Black Hole Quencher® 1, 2 and 3, Nanogold Particles®, Eclipse Dark Quencher®, Elle Quencher®, malachite green and the dyes QSY®7, QSY®9 and QSY®21.

**16.** Method according to any one of claims 8 to 15, **characterised in that** the complexes of formula (I) are selected from compounds in which:

i) (B) is selected from the peptides, proteins, oligonucleotides, sugars and peptidonucleic acids,
ii) L is fluorescein, and
iii) the skeleton of the tripod Y is selected from the following structures $Y_1$ to $Y_3$:

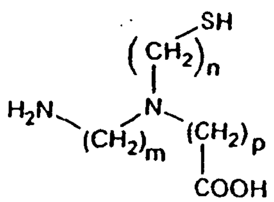
$(Y_1)$

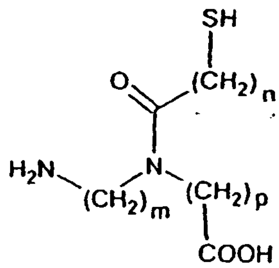
$(Y_2)$

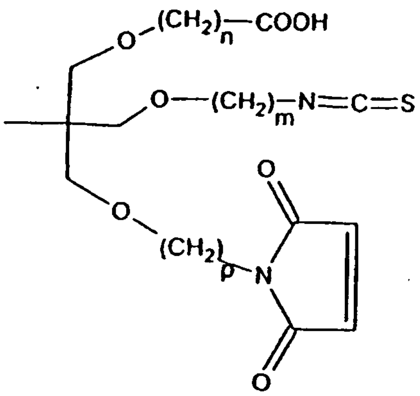
$(Y_3)$

in which n, m and p, which may be identical or different, are integers from 1 to 20 inclusive.

**17.** Method according to claim 16, **characterised in that** the structures $Y_1$ to $Y_3$ are selected from the following compounds of formulae ($Y'_1$) to ($Y'_3$):

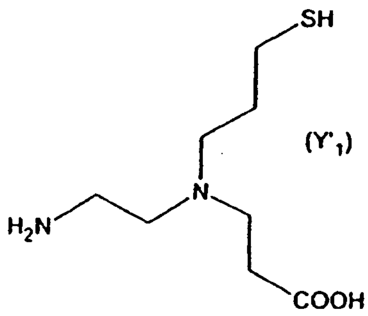

($Y'_1$)

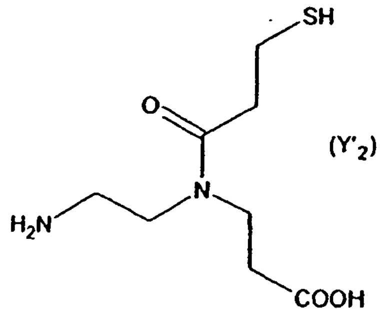

($Y'_2$)

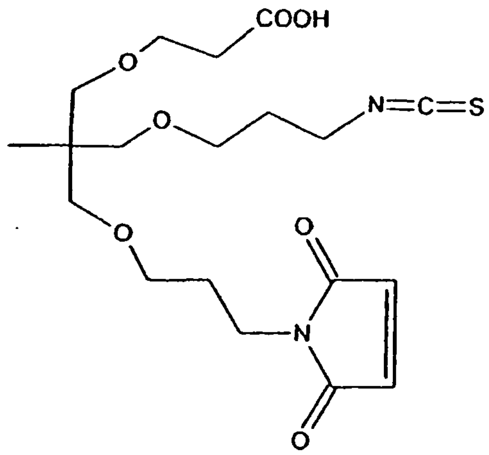

($Y'_3$)

**18.** Complexes C, **characterised in that** they correspond to the following formula (I):

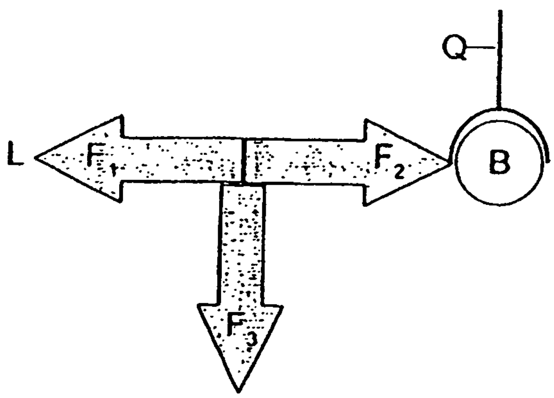


(I)

in which L, B, Q, the arrows and $F_1$, $F_2$ and $F_3$ are as defined in any one of claims 8 to 17.

**19.** Use of at least one complex C of formula (I) as defined in any one of claims 8 to 16 in a method of continuously detecting, in a heterogeneous phase, an analyte a in a liquid sample.

**20.** Device for the continuous detection, in a heterogeneous phase, of at least one analyte a in a liquid sample, said

device being **characterised in that** a liquid sample to be analysed is incorporated into a medium forming a flux which flows over at least one solid support, to the surface of which there is fixed at least one tripod Y as defined in claim 1 and specific to the analyte a to be detected, a luminescence detector arranged facing the solid support is coupled to a valve drive which is triggered at a signal intensity threshold emitted by the detector and which initiates, for a predetermined period of time, the opening of a tank containing a receptor-Q capable of forming a complex with the tripod Y, the tank being connected to the support by a feedback loop which opens upstream of the solid support to which the tripod Y is fixed, in order to saturate and/or regenerate the solid support with receptor-Q by passage into the flux and complexing on the tripod Y.

**21.** Device according to claim 20, in which the values of the intensity of the luminescence are monitored and, if necessary, converted into quantity of analyte a by a computation system coupled to the luminescence detector.

**22.** Device according to claim 20 or 21, in which an event marker is arranged in the feedback loop in order to indicate a variation in the intensity of the signal that is greater than a predetermined value.

**23.** Device according to any one of claims 20 to 22, in which the solid support is a capillary coupled to the environment containing the sample to be analysed, the coupling being effected either by way of a collecting flask in which the sample bubbles through a medium corresponding to that of the flowing flux, or by way of a flexible tube.

**24.** Device according to any one of claims 20 to 23, in which the flux is drawn along by the low pressure produced by a pump, a piston or the like.

**25.** Device according to any one of claims 20 to 24, **characterised in that** it is equipped with a collecting flask and with a bubbling system, allowing samples to be collected in gas form and the constituents to be detected contained therein to be dissolved.

**26.** Use of a device as defined in any one of claims 20 to 25 for detecting the presence of an analyte a in a natural or industrial medium.

**27.** Use according to claim 26, **characterised in that** the device is used in lakes, rivers, swimming pools, factories, sewage plants, ventilation systems or air-conditioning systems.

**Patentansprüche**

**1.** Verfahren zur Detektion eines Analyten a in einer Fluidprobe, **dadurch gekennzeichnet, dass** es die folgenden Stufen umfasst:

1) die Sättigung eines festen Trägers, der auf wenigstens einem Teil seiner Oberfläche wenigstens ein trifunktionelles Reagenz (Y-Tripode) enthält, das die drei folgenden funktionellen Pole enthält:

i) eine lumineszierende Gruppierung (L),
ii) ein Molekül (B), das unter dem Analyten a, einem Analogon des Analyten a und einem Fragment des Analyten a ausgewählt ist; und
iii) eine Funktion, die die Fixierung des trifunktionellen Reagenzes an der Oberfläche des festen Trägers sicherstellt;

durch einen Rezeptor des Analyten a, wobei der Rezeptor durch eine Akzeptorverbindung (Q) (Rezeptor-Q) der Lumineszenz der Gruppierung L markiert ist, um einen Komplex C zwischen dem Molekül (B) und dem Rezeptor-Q zu bilden;
2) Inkontaktbringen des in Stufe 1) erhaltenen festen Trägers mit einer Fluidprobe, die den zu detektierenden Analyten a enthalten könnte;
3) Messung der Intensität des Signals, das durch die Gruppierung L emittiert wird, die proportional zu der Menge an Analyt a ist, die in der Fluidprobe vorliegt; und
4) Regenerierung des festen Trägers durch Inkontaktbringen des festen Trägers mit dem Rezeptor-Q.

**2.** Verfahren nach Anspruch 1, **dadurch gekennzeichnet, dass** mehrere Typen an Y-Tripoden, die sich durch die Natur des Moleküls (B), das sie enthalten, unterscheiden, an unterschiedlichen und bekannten Zonen des festen

Trägers fixiert werden.

**3.** Verfahren nach Anspruch 1 oder 2, **dadurch gekennzeichnet, dass** die Stufen 3) und 4) kontinuierlich durch geführt werden.

**4.** Verfahren nach einem der vorangehenden Ansprüche, **dadurch gekennzeichnet, dass** der feste Träger unter Gläsern, Kunststoffen, Keramiken, Metallen und Metalloiden ausgewählt wird.

**5.** Verfahren nach einem der vorangehenden Ansprüche, **dadurch gekennzeichnet, dass** der Träger in Form eines Schlauchs, einer Kapillare, einer Platte oder einer Kugel vorliegt.

**6.** Verfahren nach einem der vorangehenden Ansprüche, **dadurch gekennzeichnet, dass** die Fluidprobe aus Wasser, einem flüssigen biologischen Medium oder einem flüssigen Medium, das Moleküle enthält, die gelöste gasförmige Moleküle sind oder von festen Proben stammen, besteht.

**7.** Verfahren nach einem der vorangehenden Ansprüche, **dadurch gekennzeichnet, dass** die Intensität des während Stufe 3 emittierten Signals durch einen Lumineszenzdetektor gemessen wird.

**8.** Verfahren nach einem der vorangehenden Ansprüche, **dadurch gekennzeichnet, dass** der in der Sättigungsstufe 1) gebildete Komplex unter den Komplexen der folgenden Formel (I) ausgewählt wird:

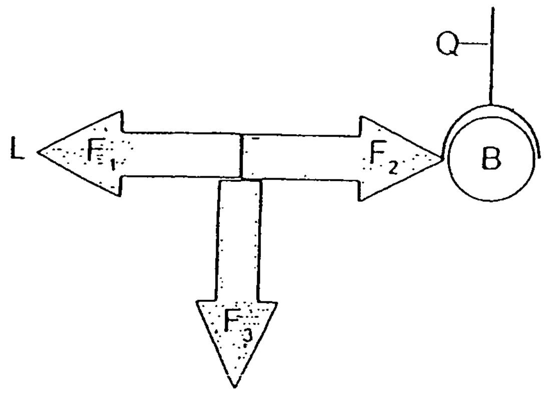


(I)

worin:

- die Pfeile die Struktur des Grundgerüsts der Y-Tripode darstellen, die ein Bindungsarm ist, der durch eine Peptid-, Nucleotid-, Glucosidkette oder durch eine Kohlenwasserstoffkette linear oder verzweigt, gesättigt oder ungesättigt, gebildet wird; wobei die Ketten gegebenenfalls substituiert, unterbrochen und/oder terminiert sind durch ein oder mehrere Heteroatome wie N, O oder S und/oder durch eine oder mehrere Aminosäuren und drei reaktive chemische Funktionen $F_1$, $F_2$ und $F_3$ enthalten;
- L eine lumineszierende Gruppierung darstellt, die über die reaktive chemische Funktion $F_1$ kovalent an die Y-Tripode gebunden ist;
- B einen Analyten a, ein Strukturanalogon eines Analyten a oder ein Fragment eines Analyten a darstellt, an dem in nicht-kovalenter und reversibler Art ein spezifischer Rezeptor des Analyten a fixiert ist, wobei der Rezeptor durch eine Verbindung Q markiert ist; wobei das Molekül (B) in kovalenter Art über die reaktive chemische Funktion $F_2$ an die Y-Tripode gebunden ist;
- Q eine Akzeptorverbindung der Lumineszenz der Gruppierung L darstellt;
- $F_3$ eine reaktive chemische Funktion darstellt, die geeignet ist, die Fixierung der Y-Tripode an der Oberfläche des festen Trägers zuzulassen.

**9.** Verfahren nach Anspruch 8, **dadurch gekennzeichnet, dass** die Funktionen $F_1$, $F_2$, $F_3$ unabhängig voneinander

i) entweder eine direkte Bindung über eine entsprechende chemische Funktion, die an der lumineszierenden Verbindung, dem Molekül (B) oder der festen Phase vorhanden ist,
ii) oder eine indirekte Bindung gewährleistet, wobei in diesem Fall die Bindung verwirklicht wird, indem wenig-

stens eine der Funktionen $F_1$, $F_2$ und/oder $F_3$, ein Molekül $M_1$, das zur Bildung eines Komplexes mit einem Molekül $M_2$, das vorher an wenigstens einem Teil der Oberfläche der festen Phase fixiert wurde, fähig ist, an das Molekül (B) und/oder an die lumineszierende Gruppe gekoppelt wird.

**10.** Verfahren nach Anspruch 8 oder 9, **dadurch gekennzeichnet, dass** die Funktionen $F_1$, $F_2$ und $F_3$, die identisch oder unterschiedlich sind, unter den Funktionen Thiole; Amine; Alkohole; Säuren; Esther; Isothiocyanate; Isocyanate; Acylacide; Sulfonylchloride; Aldehyde; Glyoxale; Epoxide; Oxirane; Carbonate; Imidoester; Carbodiimide; Maleimide; Nitrile, Aziridine; Acryloyl; halogenierte Derivate; den Disulfidgruppierungen; Phosphorgruppierungen; Diazogruppierungen; Carbonyldümidazolgruppierungen; Hydrazidgruppierungen; Arylacidgruppierungen; Hydrazingruppierungen; Diaziringruppierungen; Magnesiumgruppierungen; Lithiumgruppierungen; Kupfergruppierungen; Zinkgruppierungen und den ungesättigten Systemen ausgewählt sind.

**11.** Verfahren nach Anspruch 10, **dadurch gekennzeichnet, dass** die Funktionen $F_1$, $F_2$ und $F_3$ unter den Aminfunktionen der Formel $R\text{-}NH_2$, R-NH-, $(R)_3\text{-}N$, R-NH-OR und $NH_2\text{-}OR$; den Alkoholfunktionen R-OH und den halogenierten Gruppierungen der Formel R-X, worin X ein Halogenatom darstellt, ausgewählt sind, wobei R in den genannten Formeln einen Alkyl-, Aryl-, Vinyl- oder Allylrest darstellt.

**12.** Verfahren nach einem der vorangehenden Ansprüche, **dadurch gekennzeichnet, dass** die lumineszierenden Gruppierungen unter Fluorescein (Fluoresceinnatrium) und seinen Derivaten; Rhodamin und seinen Derivaten, Diaminidophenylindo (DAPI); Acridin; fluoreszierenden reaktiven Aminfarbstoffen; den Fluoreszenzfarbstoffen, die unter den Handelsbezeichnungen BODIPY® verkauft werden; den Cascade Blue-Farbstoffen, Cy2, Cy3, Cy3.5, Cy5, Cy5.5, Cy7, DABCYL® und EDANS®; Eosin; Erythrosin; 6-Fam und Texas Rot ausgewählt werden.

**13.** Verfahren nach einem der vorangehenden Ansprüche, **dadurch gekennzeichnet, dass** die Rezeptoren unter den Antikörpern in ganzer Form, Fragmentform oder rekombinanter Form, biologischen Rezeptoren, Nucleinsäuren, Peptidonucleinsäuren, Lectinen, Transportproteinen, Chelaten und synthetischen Rezeptoren ausgewählt werden.

**14.** Verfahren nach einem der vorangehenden Ansprüche, **dadurch gekennzeichnet, dass** der Rezeptor eine größere Affinität für den Analyten a als für das Molekül (B) zeigt.

**15.** Verfahren nach einem der vorangehenden Ansprüche, **dadurch gekennzeichnet, dass** die Akzeptorverbindung (Q) unter Rhodamin und seinen Derivaten, den in Anspruch 12 genannten fluoreszierenden Verbindungen, nichtfluoreszierenden Molekülen, ausgewählt unter den Verbindungen, die unter den Handelsbezeichnungen Black Hole Quencher® 1, 2 und 3, Nanogold Particules®, Eclipse Dark Quencher®, Elle Quencher® verkauft werden, Malachitgrün und den Färbemitteln QSY®7, QSY®9 und QSY®21 ausgewählt werden.

**16.** Verfahren nach einem der Ansprüche 8 bis 15, **dadurch gekennzeichnet, dass** die Komplexe der Formel (I) unter den Verbindungen ausgewählt werden, in denen:

i) (B) aus Peptiden, Proteinen, Oligonucleotiden, Zuckern und Peptidonucleinsäuren ausgewählt wird,
ii) L Fluorescein ist und
iii) das Grundgerüst der Y-Tripode unter den folgenden Strukturen $Y_1$ bis $Y_3$ ausgewählt ist:

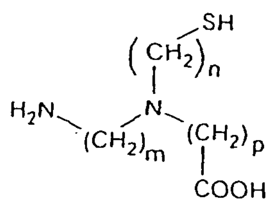 $(Y_1)$

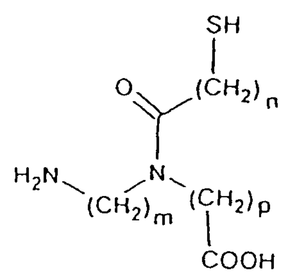 $(Y_2)$

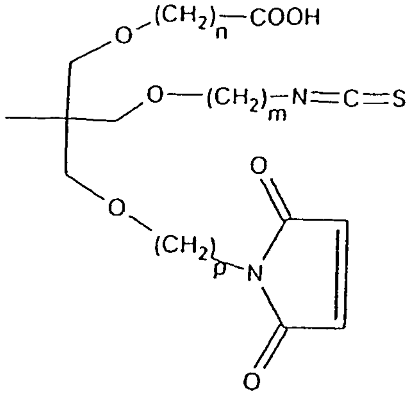

(Y$_3$)

worin n, m und p, die identisch oder verschieden sind, ganze Zahlen zwischen 1 und 20, einschließlich, sind.

**17.** Verfahren nach Anspruch 16, **dadurch gekennzeichnet, dass** die Strukturen $Y_1$ bis $Y_3$ unter den folgenden Verbindungen der Formeln (Y'$_1$) bis (Y'$_3$) ausgewählt werden:

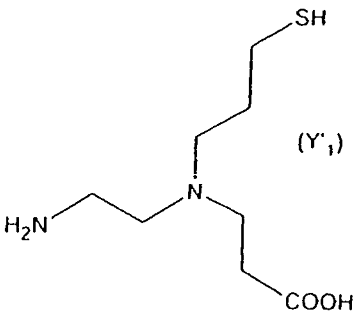

(Y'$_1$)

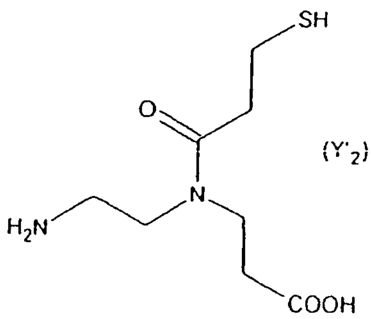

(Y'$_2$)

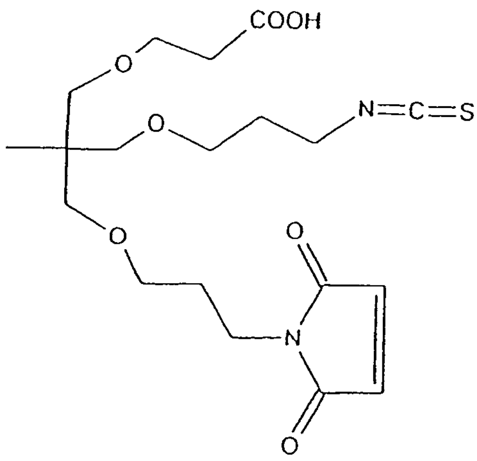

(Y'$_3$)

**18.** Komplexe C, **dadurch gekennzeichnet, dass** sie der folgenden Formel (I) entsprechen:

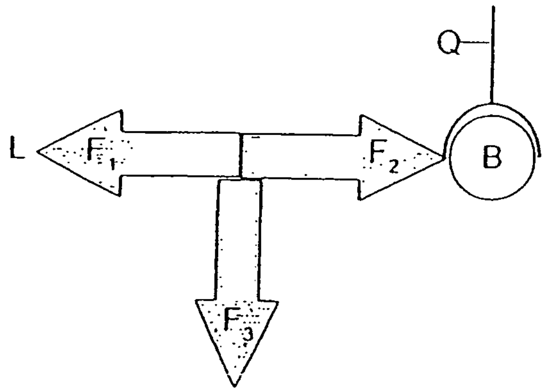


(I)

in der L, B, Q, die Pfeile und $F_1$, $F_2$ und $F_3$ wie in einem der Ansprüche 8 bis 17 definiert sind.

**19.** Verwendung wenigstens eines Komplexes C der Formel (I) wie in einem der Ansprüche 8 bis 16 definiert in einem Verfahren zur kontinuierlichen Detektion in heterogener Phase eines Analyten a in einer Fluidprobe.

**20.** Vorrichtung zur kontinuierlichen Detektion in heterogener Phase wenigstens eines Analyten a in einer Fluidprobe, wobei die Vorrichtung **dadurch gekennzeichnet ist, dass** eine zu analysierende Fluidprobe in ein Medium integriert wird, das einen Strom bildet, der auf wenigstens einem festen Träger fließt, auf dessen Oberfläche wenigstens eine Y-Tripode, wie sie in Anspruch 1 definiert ist und die für den zu detektierenden Analyten a spezifisch ist, fixiert ist, ein Lumineszenzdetektor, der gegenüber dem festen Träger angeordnet ist, mit einem Schieberegler verbunden ist, der durch ein Schwellenintensitätssignal gesteuert wird, welches durch den Detektor emitiert wird, und der während einer bestimmten Dauer die Öffnung eines Vorratsbehälters, der einen Rezeptor-Q, der zur Bildung eines Komplexes mit der Y-Tripode im Stande ist, enthält, wobei dieser Vorratsbehälter mit dem festen Träger über eine Rückführungsschleife verbunden ist, die stromauf wärts des festen Trägers, auf dem die Y-Tripode fixiert ist, einmündet, um diesen letztgenannten an Rezeptor-Q durch Passage im Strom und Komplexbildung an der Y-Tripode zu sättigen und/oder zu regenerieren.

**21.** Vorrichtung nach Anspruch 20, in der die Werte für die Intensität der Lumineszenz überwacht und gegebenenfalls durch ein Rechnersystem, das an den Lumineszenzdetektor angeschlossen ist, in die Menge an Analyt a umgewandelt werden.

**22.** Vorrichtung nach Anspruch 20 oder 21, in der ein eigenes Markierungsgerät in der Rückführungsschleife angeordnet ist, um eine Veränderung der Intensität des Signals, die über einem vorbestimmten Wert liegt, zu signalisieren.

**23.** Vorrichtung nach einem Ansprüche 20 bis 22, in der der feste Träger eine Kapillare ist, die mit der Umgebung, die die zu analysierende Probe enthält, verbunden ist, wobei die Verbindung entweder mit Hilfe eines Fangballons, in dem die Probe in ein Medium perlt, das dem des Fließstroms entspricht, oder mit Hilfe einer elastischen Leitung verwirklicht wird.

**24.** Vorrichtung nach einem der Ansprüche 20 bis 23, in der der Strom durch den Unterdruck, der durch eine Pumpe, einen Kolben oder ein Äquivalent erzeugt wird, gezogen wird.

**25.** Vorrichtung nach einem der Ansprüche 20 bis 24, **dadurch gekennzeichnet, dass** sie mit einem Fangballon und einem Durchperlsystem bzw. Blasesystem ausgestattet ist, die es ermöglichen Proben in Gasform zu sammeln und die zu detektierenden Bestandteile, die sie enthalten, zu solubilisieren.

**26.** Verwendung einer Vorrichtung wie sie einem der Ansprüche 20 bis 25 definiert ist, um das Vorliegen eines Analyten a in einem natürlichen oder industriellen Medium zu detektieren.

**27.** Verwendung nach Anspruch 26, **dadurch gekennzeichnet, dass** die Vorrichtung in Seen, Flüssen, Schwimmbädern, Fabriken, Reinigungsstationen, Belüftungs- oder Klimatisierungssystemen verwendet wird.

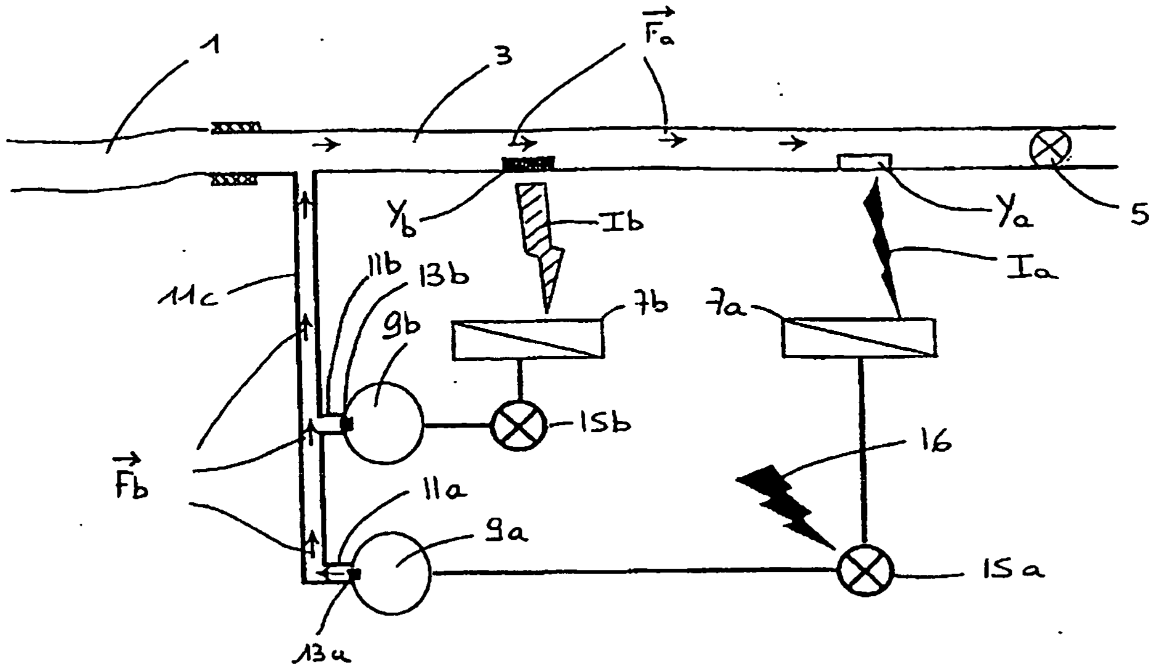


FIGURE 1

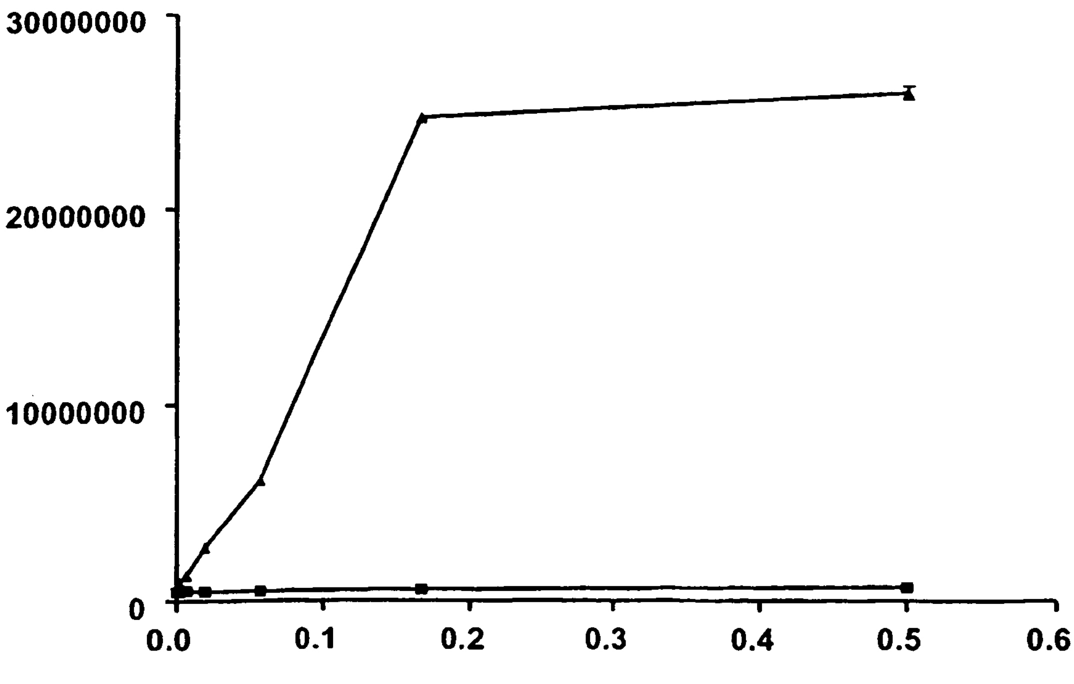


FIGURE 2

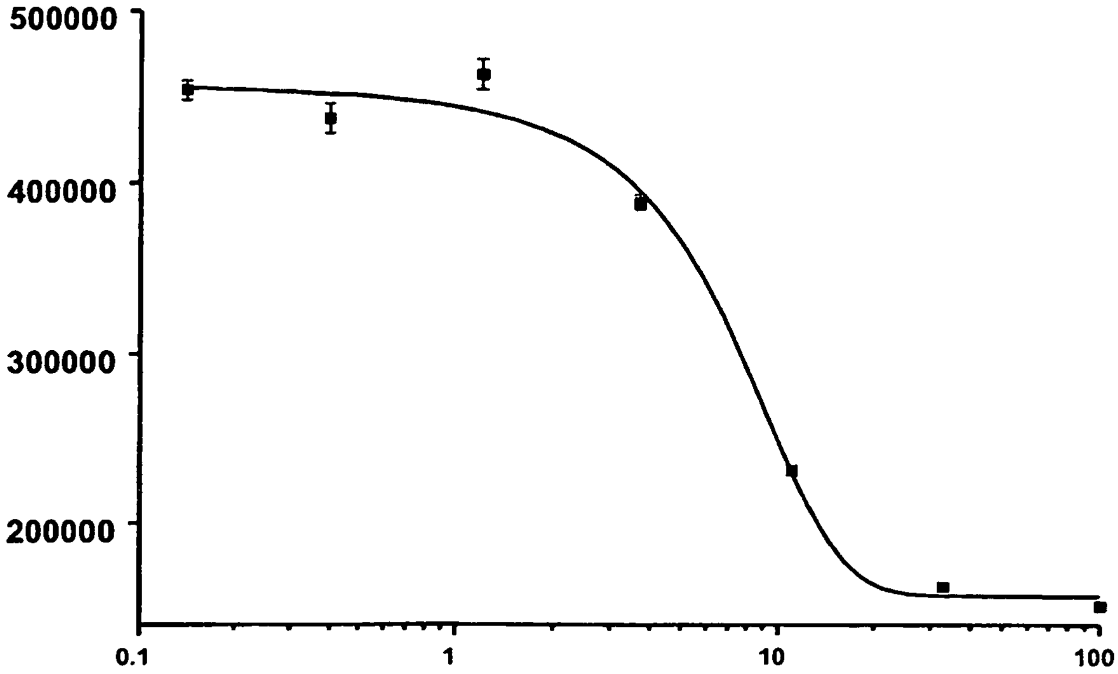


FIGURE 3

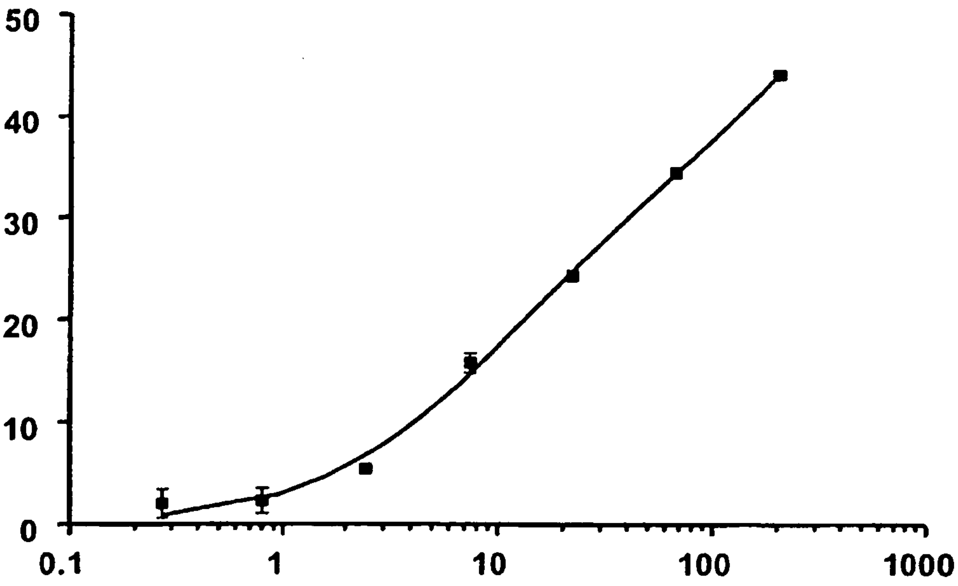


FIGURE 4

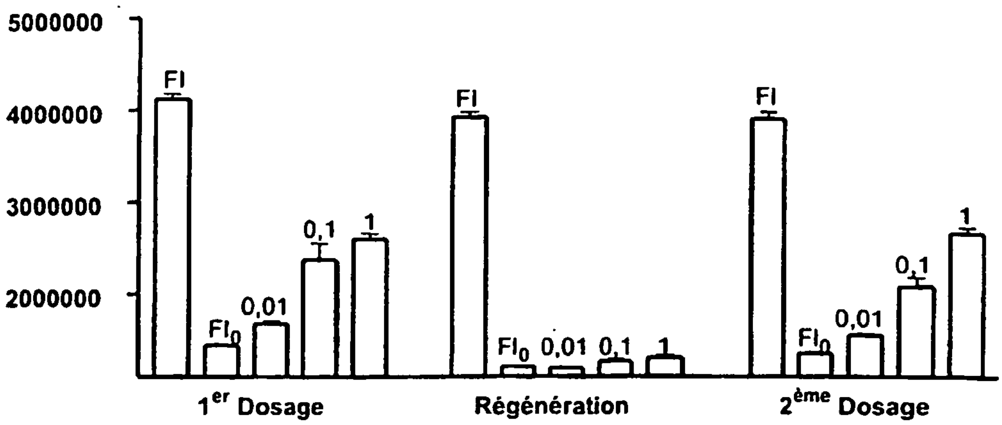


FIGURE 5

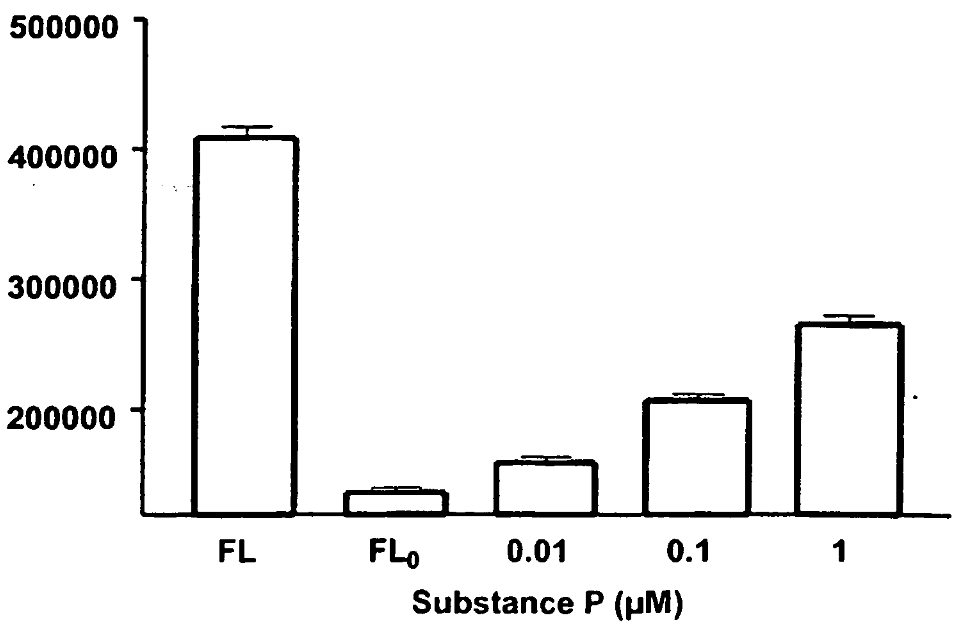


FIGURE 6

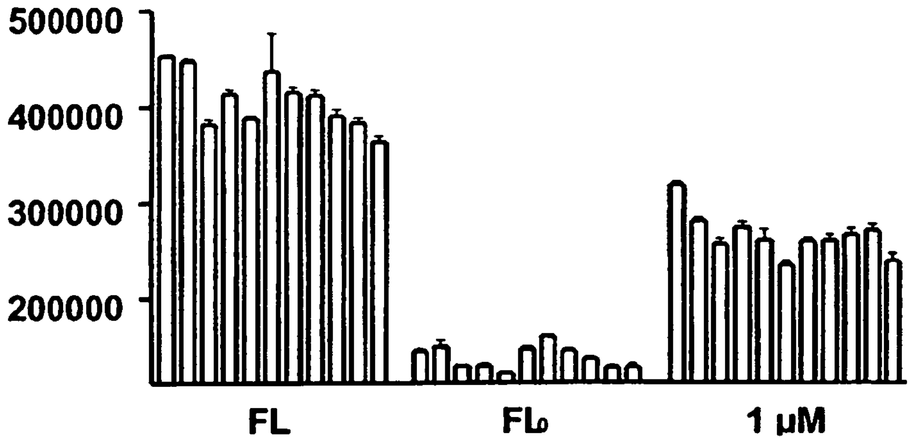


FIGURE 7

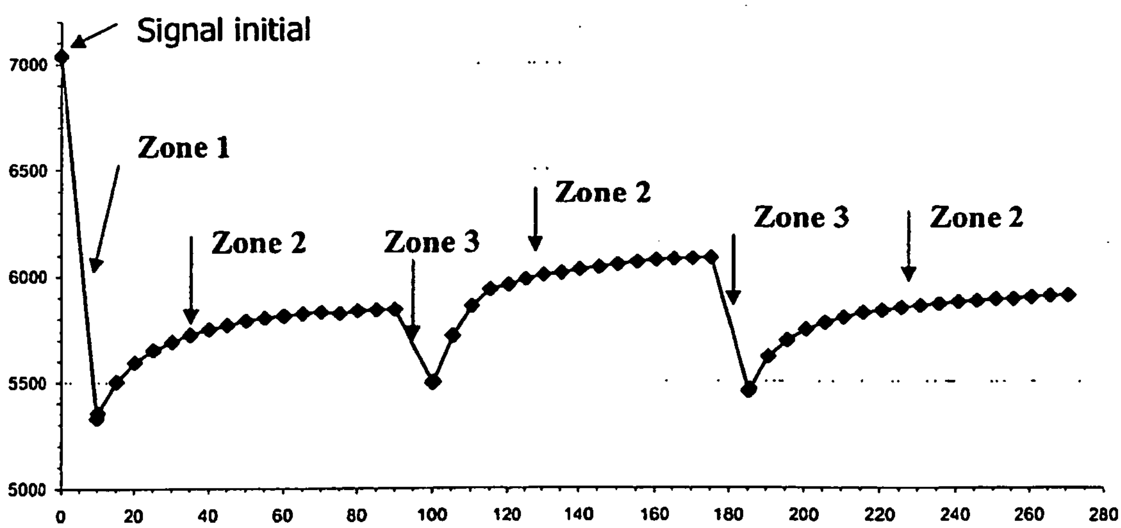


FIGURE 8